# FASCICULE DE BREVET EUROPEEN

(11) **EP 1 007 558 B1**
(45) Date de publication et mention de la délivrance du brevet: **23.04.2003**
(21) Numéro de dépôt: 98942740.6
(22) Date de dépôt: 13.08.1998
(51) Int. Cl.: C07K 14/47, C12N 15/12, C12N 15/11, C12Q 1/68

(54) **GENE DE LA FIEVRE MEDITERRANEENNE FAMILIALE**
GEN DES FAMILIÄREN MITTELMEERFIEBERS
FAMILIAL MEDITERRANEAN FEVER GENE

(30) Priorité: 19.08.1997 FR 9710487
(43) Date de publication de la demande: 14.06.2000
(73) Titulaire: Genethon III, 91002 Evry Cedex (FR)
(72) Inventeur: BERNOT, Alain, F-75005 Paris (FR); CLEPET, Christian, F-75013 Paris (FR); HEILIG, Roland, F-67000 Strasbourg (FR); WEISSENBACH, Jean, F-75015 Paris (FR); TOUITOU, Isabelle, F-34000 Montpellier (FR)
(74) Mandataire: Palix, Stéphane
(86) Numéro de dépôt international: FR9801805
(87) Numéro de publication internationale: WO99009059

(56) Documents cités:
- EP-A- 0 497 527
- THE FRENCH CONSORTIUM: "Localization of the Familial Mediterranean Fever gene (FMF) to a 250-kb interval in non-Ashkenazi Jewish founder haplotypes" THE AMERICAN JOURNAL OF HUMAN GENETICS, vol. 59, no. 3, septembre 1996, pages 603-612, XP002066931 CHICAGO, ILL, US cité dans la demande
- E.N. LEVY ET AL.,: "Linkage desequilibrium mapping places the gene causing Familial Mediterranean Fever close to D16S246" THE AMERICAN JOURNAL OF HUMAN GENETICS, vol. 58, no. 3, mars 1996, pages 523-534, XP002066932 CHICAGO, ILL, US cité dans la demande
- R. SOOD ET AL.,: "Construction of a 1-Mb restriction-mapped cosmid contig containing the candidate region for the Familial Mediterranean Fever Locus (MEFV) on chromosome 16p13.3" GENOMICS, vol. 42, no. 1, 15 mai 1997, pages 83-95, XP002066933 SAN DIEGO, CA, US cité dans la demande
- J.E. BALOW ET AL.,: "A high-resolution genetic map of the Familial Mediterranean Fever candidate region allows identification of haplotype-sharing among ethnic groups" GENOMICS, vol. 44, no. 3, 15 septembre 1997, pages 280-291, XP002066934 SAN DIEGO, CA, US cité dans la demande
- FRENCH FMF CONSORTIUM: "A candidate gene for Familial Mediterranean Fever" NATURE GENETICS, vol. 17, no. 1, 1 septembre 1997, pages 25-31, XP002066935
- THE INTERNATIONAL FMF CONSORTIUM: "Ancient missense mutations in a new member of the RoRet gene family are likely to cause familial Mediterranean fever" CELL, vol. 90, no. 4, 22 août 1997, pages 797-807, XP002066936 CAMBRIDGE, MA, US
- M. PRAS: "Familial Mediterranean fever: from the clinical syndrome to the cloning of the pyrin gene" SCANDINAVIAN JOURNAL OF RHEUMATOLOGY, vol. 27, no. 2, 1998, pages 92-97, XP002067986 OSLO, NO
- A. BERNOT ET AL., : "A TRANSCRIPTIONAL MAP OF THE FMF REGION" GENOMICS, vol. 50, 1 juin 1998, pages 147-160, XP002090566
- A. BERNOT ET AL., : "Non-founder mutations in the MEFV gene establish this gene as the cause of familial Mediterranean fever (FMF)" HUMAN MOLECULAR GENETICS, vol. 7, no. 8, août 1998, pages 1317-1325, XP002090567 GB
- X. CHEN ET AL., : "Assessment of pyrin mutations in Turks with familial Mediterranean fever (FMF)" HUMAN MUTATION, vol. 11, no. 6, 1998, pages 465-460, XP002090568 US

## Description

La présente invention concerne le domaine de la génétique. Elle concerne plus particulièrement de nouveaux acides nucléiques, notamment le gène responsable de la Fièvre Méditerranéenne Familiale (FMF), ainsi que la protéine correspondante. Elle concerne en outre des sondes et amorces pour la détection et l'amplification de ce gène, ainsi que des trousses et procédés de dépistage de la FMF. Elle concerne également toute utilisation du gène et de la protéine, notamment à des fins thérapeutiques.

La Fièvre Méditerranéenne Familiale (FMF pour "Familial Mediterranean Fever") est une maladie récessive transmissible qui affecte principalement les populations entourant la mer Méditerranée. Ainsi, cette pathologie affecte notamment les populations juives d'Afrique du nord, arméniennes, turques et arabes, chez lesquelles la fréquence de la maladie est de l'ordre de 1/250. La maladie se caractérise essentiellement par une inflammation récurrente au niveau abdominal et pulmonaire (notamment du péritoine, de la synoviale et de la plèvre). En outre, des complications rénales importantes sont observées en l'absence de traitement efficace.

Actuellement, les symptômes de cette maladie peuvent être réduits de manière satisfaisante par un traitement à la colchicine, lorsqu'elle est prise en charge suffisamment tôt. Néanmoins, il n'existe pas aujourd'hui de méthode de dépistage ou de diagnostic efficace de cette pathologie. Aussi, la mise à disposition de telles méthodes permettrait une prise en charge très précoce de la pathologie, et éviterait ainsi les complications rénales qui sont bien plus handicapantes pour les sujets atteints, et difficiles à soigner. En outre, l'identification du gène et des mécanismes biochimiques à la base de cette pathologie pourrait aussi permettre le développement de nouvelles approches thérapeutiques plus efficaces.

Différentes études ont été réalisées dans l'art antérieur pour tenter d'élucider les altérations génétiques impliquées dans la FMF. Dans la mesure où la déficience biochimique impliquée dans la FMF est inconnue, une étude de clonage positionnel a été entreprise pour tenter de localiser le gène FMF. Ainsi, un gène candidat responsable de la FMF (désigné gène MEFV) a été initialement localisé sur le bras court du chromosome 16, initialement sur une région de 9 centimorgans, puis ensuite sur une région de 4 centimorgans, entre les marqueurs D16S246 et D16S523 (ref 4). Par des études de déséquilibres de liaisons et d'analyse d'haplotypes, un intervalle de 250 kb a par la suite été défini, entre les marqueurs D16S3070 et D16S3275 (ref 5). Au cours de ces études, différents haplotypes fondateurs ont ainsi été identifiés dans différents groupes ethniques, tels que présentés dans le Tableau 1 ci-dessous :

**Tableau 1**

| Haplotype | Groupe Ethnique |
|---|---|
| S (MED) | Juifs d'Afrique du Nord |
| ARM1 (MED) | Arméniens |
| ARM2 | Arméniens |
| ARM3 | Arméniens |
| T (MED) | Turcs |
| ARA1 (MED) | Arabes du Maghreb |
| ARA2 | Arabes du Maghreb |
| D | Druzes |

En outre, ces études ont également suggéré que les haplotypes S, ARM1, T et ARA1 présentent une origine ancestrale commune, l'haplotype fondateur méditerrannéen désigné MED (Cf tableau ci-dessus). La fréquence de ces altérations génétiques dans les populations mentionnées ci-dessus est très élevée, le taux de sujets porteurs atteignant 1/6 dans la population juive d'Afrique du nord (ref 1) et 1/7 dans la population arménienne (ref 2).

En dépit de ces études, il n'a cependant pas été possible jusqu'à présent d'identifier et de caractériser la ou les altérations génétiques précises responsables de la FMF. En particulier, aucun gène n'a été identifié ni aucune protéine correspondante, et le type d'altérations génétiques impliquées dans cette pathologie demeure inconnu.

La présente invention résulte de la découverte et de l'identification par les inventeurs du gène responsable de la FMF. La présente invention résulte également de la caractérisation de plusieurs mutations sur ce gène, qui sont présentes uniquement chez les sujets atteints de la FMF. La présente demande décrit également des sondes nucléiques, spécifiques du gène FMF normal ou muté, permettant la détection de la présence ou non de mutations chez des sujets. L'invention est encore relative à un procédé et un kit de détection de la FMF comprenant des sondes spécifiques de ce gène, et éventuellement des amorces spécifiques pour son amplification. Par ailleurs, la présente demande fournit également une nouvelle stratégie de thérapie de la FMF, basée sur l'utilisation de la protéine ou du gène natif. L'utilisation peut être soit directe, pour restaurer l'activité correspondante chez les sujets, ou indirecte, en utilisant ces matériels comme cibles pour la mise au point de médicaments plus efficaces (modélisation moléculaire, chimie combinatoire, recherche de partenaires, etc.).

Un premier aspect de l'invention réside donc plus particulièrement dans la mise à disposition de nouveaux acides nucléiques. Ainsi, un premier objet de l'invention concerne un acide nucléique isolé codant pour la séquence d'acide aminés correspondant à la séquence SEQ ID 1.

Un objet de l'invention réside également dans un acide nucléique comprenant une séquence codant pour tout ou partie de la protéine dont la forme mutée est responsable de la Fièvre Méditerranéenne Familiale (FMF).

Le terme "acide nucléique" désigne au sens de l'invention tout acide désoxyribonucléique (ADN) ou ribonucléique (ARN). Un ADN peut être plus particulièrement un ADN complémentaire (ADNc), un ADN génomique (ADNg) ou un ADN synthétique, comprenant par exemple un ou plusieurs des introns de l'ADN génomique. L'ARN peut être préférentiellement un ARN messager (ARNm). Les acides nucléiques de l'invention peuvent être d'origine diverses, et notamment d'origine mammifère, synthétique ou semi-synthétique. Ils peuvent être isolés par toutes les techniques connues de la Biologie moléculaire, en utilisant les données structurales et de séquences fournies par la présente demande. Ainsi, ces acides nucléiques peuvent être isolés à partir de banques, par des techniques d'hybridation. Ils peuvent aussi être synthétisés chimiquement ou génétiquement.

Pour identifier le gène responsable de la FMF, la demanderesse a réalisé le séquençage complet d'une région candidate de 250 kb à partir d'un contig minimal, consistant en 8 clones de cosmides et un fragment de 3 kb obtenu par PCR.

Des études appronfondies de polymorphisme ont ensuite été effectuées sur des SNP ("Single Nucleotide Polymorphism") nouvellement générés à partir de la séquence de l'intervalle D16S3070-D16S3275, et ont permis de confirmer l'existence d'un haplotype fondateur commun au sein de différentes populations arméniennes, turques, juives et arabes. Ces marqueurs ont également permis de confirmer l'existence d'événements de recombinaison dans l'haplotype fondateur, et ont ainsi permis de réduire le locus candidat à une région de 60 kb, délimitée par les marqueurs D16S2617 et AFMef101-D16S3373.

En effectuant des analyses de séquences génomiques et des expériences d'exon-trapping, plusieurs unités transcriptionnelles ont pu être identifiées dans cet intervalle de 60 kb. Ces méthodes d'analyse ont été utilisées pour plusieurs raisons, et notamment par ce qu'elles sont adaptées à des séquences génomiques et sont indépendantes de l'expression (la méthode d'exon-trapping repose sur les mécanismes d'épissage dans les cellules vivantes, et les analyses par séquençage reposent principalement sur l'utilisation de programmes informatiques). La validation des prédictions d'exons a conduit à la caractérisation, dans cet intervalle, de trois gènes:
- un gène désigné Olfmf, codant un récepteur olfactif;
- un gène désigné Znfmf, codant une protéine présentant des homologies avec les protéines à doigt-de-zinc et,
- un gène codant une protéine désignée Marenostrine, constituant un nouveau membre d'une famille de protéines comprenant la RFP (ref 17), la BT (ref 18), la Pwa33 (ref 19), la RPT-1 (ref 20) et la SS-A (ref 21).
Un quatrième gène a été caractérisé seulement par alignement avec des ESTs.

L'approche exhaustive utilisée pour l'identification de ces gènes rend très improbable la présence d'autres gènes non-identifiés dans l'intervalle étudié.

Afin de déterminer parmi les gènes identifiés ci-dessus, celui impliqué dans la FMF, des études de variations de séquences ont été réalisées, en comparant l'ADN de sept patients homozygotes pour un des haplotypes fondateurs à onze echantillons contrôles. Pour analyser la région codante de ces gènes, la technique de séquençage de produits PCR d'ADN génomique a été utilisée. Cette technique a permis d'identifier 4 mutations dans trois codons différents du dernier exon du gène de la Marenostrine. Plusieurs types de preuves confirment l'implication de ce gène dans la pathogénèse de la FMF :
(i) il existe une corrélation parfaite entre les phénotypes affectés observés entre les générations et les altérations dans ce gène;
(ii) aucune de ces modifications génétiques (mutations de séquences) n'a été retrouvée dans l'ADN de sujets sains ni dans l'ADN d'autres sujets non porteurs mais présentant des analogies chromosomiques avec les haplotypes fondateurs;
(iii) aucune des autres variations de séquences observées dans les autres gènes présents dans l'intervalle de 60 kb n'a montré d'association avec la pathologie.

Plus particulièrement, les mutations observées dans le gène de la Marenostrine sont représentées dans le Tableau 2 de l'exemple 7.

Il est intéressant de noter que les altérations de séquences observées résultent toutes dans un changement conservatif d'acides aminés hydrophobes. Bien que ce type de changement d'acides aminés ait été décrit le plus souvent comme n'ayant que peu
ou pas d'effet phénotypique, ses conséquences peuvent être bien plus dramatiques. Ainsi, les mutations les plus importantes dans le gène de la transthyrétine, résultant en une polyneuropathie amyloide, sont des substitutions Val -> Met (30), Leu -> Met (111) et Val -> Ile (122) (ref 25). Il est à noter à cet égard que tout comme pour la FMF, ces mutations produisent une amyloidose, même s'il s'agit dans le cas de la transthyrétine d'un syndrome primaire, alors qu'il s'agit d'un syndrome secondaire pour la FMF. Plusieurs mécanismes, tels que la modification de la conformation ou de la stabilité par exemple, l'altération d'un site de liaison ou d'un autre site actif, pourraient rendre compte des effets phénotypiques de ce type de mutations.

Un autre élément remarquable réside dans la localisation de ces 4 mutations, qui sont toutes situées dans le même exon du gène de la marenostrine.

Le tableau 2 montre la présence de la même mutation (ATG/GTG -> Met/Val) dans différentes familles présentant l'haplotype fondateur MED (S, ARM1, T ou ARA1), indiquant que cet événement de mutation est vraisemblablement très ancien, et confirmant l'existence d'un effet fondateur à l'origine d'une grande fraction des cas de FMF dans le bassin méditerranéen. La présence d'une mutation unique (GTT/GCT -> Val/Ala) commune aux haplotypes ARM3 et D, qui partagent par ailleurs d'autres allèles environnants, indique également que ces chromosomes sont issus d'un haplotype commun. La présence de mutations différentes dans les autres haplotypes fondateurs confirme par ailleurs les origines distinctes des chromosomes correspondants.

L'ensemble de ces éléments montre clairement que le gène de la Marenostrine est le seul gène candidat présent dans l'intervalle MEFV, présentant des variations de séquences strictement associées à la maladie. Par ailleurs, les résultats obtenus montrent que ces mutations sont présentes dans 85% des chromosomes porteurs, mais n'ont jamais été trouvées sur un total de 308 chromosomes controles testés, parmi lesquels 162 chromosomes non porteurs validés. Il peut donc être conclu que le gène de la Marenostrine est le gène MEFV, et que les mutations décrites ci-dessus, qui sont spécifiques des chromosomes porteurs, sont responsables de la maladie FMF.

Ainsi, comme indiqué ci-avant, un objet de l'invention réside dans un acide nucléique comprenant une séquence codant tout ou partie de la protéine dont la forme mutée est responsable de la Fièvre Méditerranéenne Familiale (FMF).

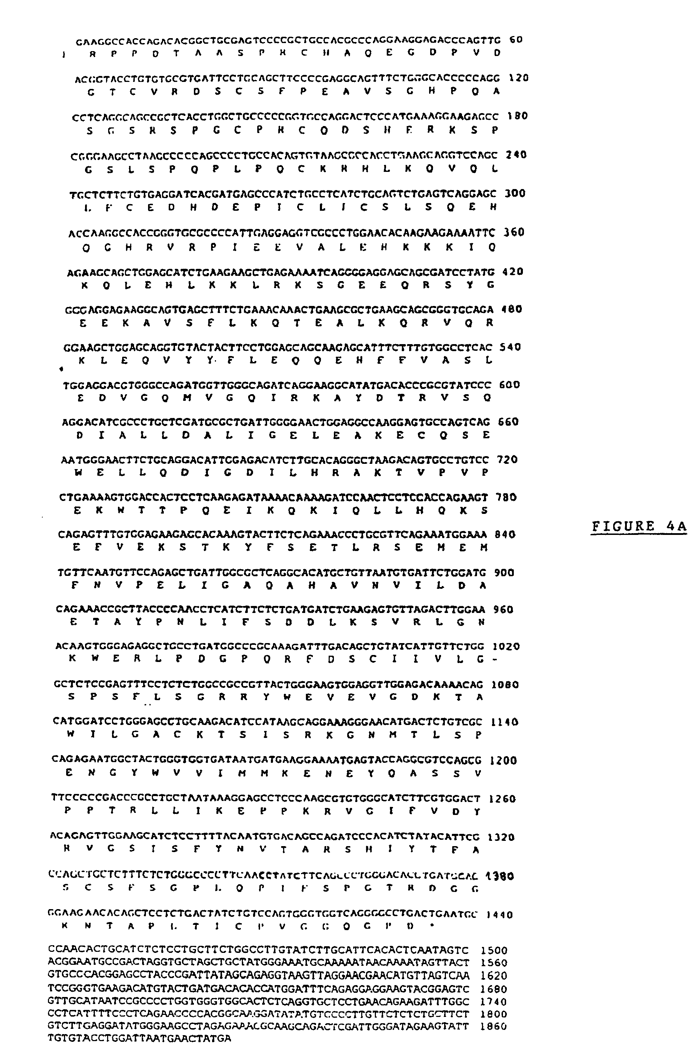

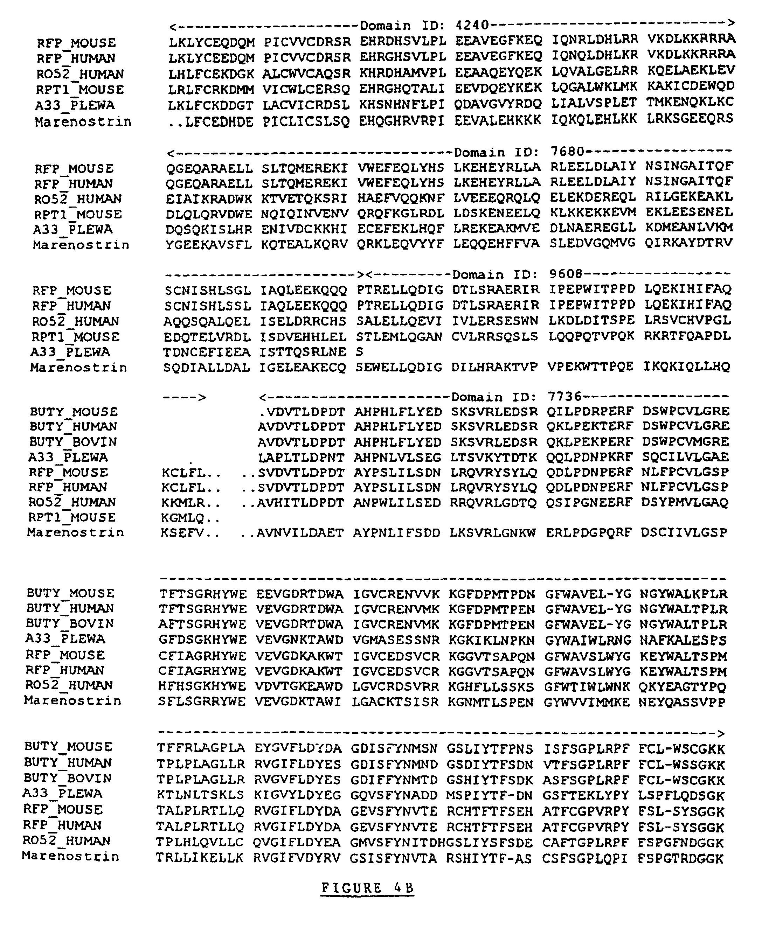

Un objet plus particulier de l'invention réside dans un acide nucléique isolé codant pour la séquence d'acides aminés correspondant à la séquence SEQ ID1 (Figure 4A), caractérisé en ce qu'il code la marenostrine.

Un exemple particulier d'acide nucléique selon l'invention est un ADNc comportant la séquence SEQ n° 1.

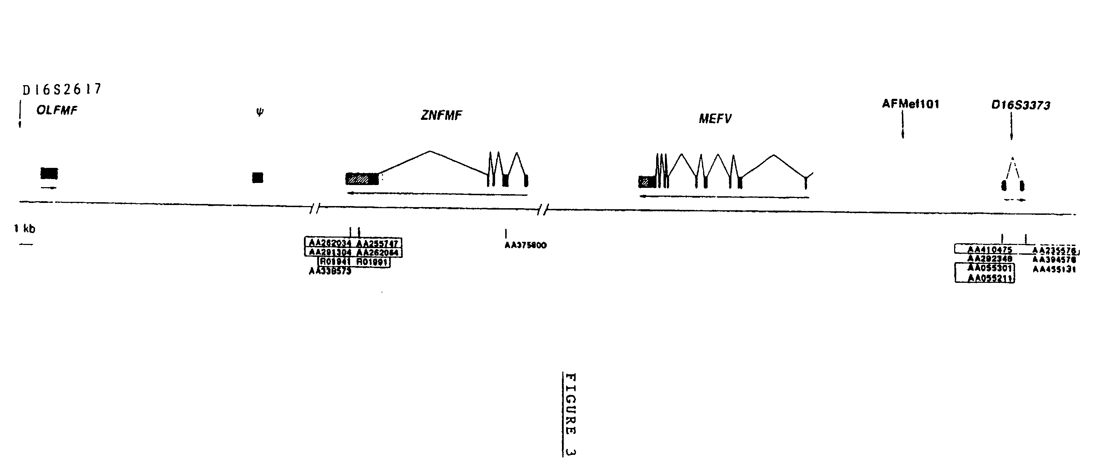

Un autre exemple particulier d'acide nucléique selon l'invention est constitué du gène humain comprenant la séquence SEQ ID 1 représentée sur la figure 4A (SEQ ID n° 1). Une carte de ce gène est également donnée sur la Figure 3.

Encore un autre exemple particulier d'acides nucléiques selon l'invention est représenté par la séquence SEQ ID n° 1 portant une ou plusieurs des mutations suivantes :
- A -> G (1170)
- G -> A (1172)
- T -> C (1267)
- G -> C (1130)

D'autres exemples d'acides nucléiques de l'invention sont donnés dans ce qui suit et dans la partie expérimentale.

Compte tenu de la fréquence importante de l'altération de ce gène dans les populations considérées, l'identification de ce gène offre de nouvelles applications diagnostiques et thérapeutiques importantes.

Un autre aspect de la présente demande est plus particulièrement relatif à des sondes et amorces spécifiques du gène ci-dessus identifié, ainsi qu'à un procédé et kit pour la détection et/ou l'amplification de ce gène. En particulier, l'invention est relative à des procédés permettant de détecter la présence de mutation(s) dans le gène ci-dessus.

Un objet particulier de l'invention concerne donc une sonde, permettant la détection du gène de la Marenostrine correspondant à tout ou partie de la séquence SEQ ID 1 ou de son brin complémentaire.

On entend par sonde au sens de l'invention, un acide nucléique simple-brin complémentaire d'un acide nucléique cible, et permettant par hybridation de détecter la présence dudit acide nucléique cible dans un échantillon.

Avantageusement, la sonde de l'invention comprend au moins une partie de la séquence SEQ ID n° 1. Au moins une partie signifie au moins 5 bases consécutives, de préférence au moins 10, encore plus préférentiellement au moins 20. Il peut s'agir d'une partie interne de la séquence concernée (notamment la SEQ ID n° 1), ou d'une extrémité de celle-ci. Dans cette dernière hypothèse, la sonde peut comprendre en outre les séquences génomiques flanquantes de la séquence concernée (notamment la SEQ ID n° 1) notamment les séquences génomiques du gène de la Marenostrine.

La sonde selon l'invention peut comprendre avantageusement de 20 à 1000 nucléotides, de préférence de 50 à 800. Plus préférentiellement, les sondes de l'invention comprennent de 100 à 700 nucléotides. encore plus préférentiellement de 100 à 500 nucléotides.

Les sondes de l'invention permettent avantageusement de détecter de manière sélective les acides nucléiques définis ci-dessus, notamment le gène de la Manenostrine. Pour cela, les sondes de l'invention sont capables d'hybrider de manière spécifique avec ces acides nucléiques, de préférence avec le gène de la Marenostrine.

Les conditions d'hybridation sont les conditions standard connues de l'homme du métier. Il peut s'agir de conditions stringentes, telles que notamment : Hybridation à 42°C, 50% formamide, 5 X SSC, 1 X Denhardt ; Lavage à 65°C en 0,1 X SSC, 0.1% SDS. D'autres conditions d'hybridation particulières sont données dans les exemples et matériels et méthodes (voir aussi Maniatis).

Les conditions stringentes sont particulièrement adaptées lorsque l'acide nucléique test est présent en faibles quantités et/ou sous forme peu purifiée. Les conditions stringentes permettent d'assurer la sélectivité de la réaction d'hybridation, c'est-à-dire de diminuer significativement le bruit de fond (hybridation avec des séquences autres que celles définies ci-dessus)

L'hybridation peut également être réalisée en conditions non-stringentes, et notamment : Hybridation à 37°C, 40% formamide, 5 X SSC, 1 X Denhardt ; Lavage à 50°C en 1 X SSC, 0,1% SDS. D'autres conditions d'hybridation particulières sont données dans les exemples et matériels et méthodes.

Ces conditions non stringentes sont plus adaptées lorsque l'acide nucléique test est présent en quantités plus importantes et se trouve sous forme significativement représentée dans l'échantillon test.

Une sonde avantageuse au sens de l'invention est composée de 100 à 700 nucléotides, dont la moitié au moins est représentée par un fragment de la séquence SEQ ID n°1. Lorsque toute la sonde de l'invention n'est pas représentée par un fragment de la séquence SEQ ID n°1, le reste est représenté par la séquence flanquante de la séquence SEQ ID 1 s'étendant depuis le résidu 1433, ou son brin complémentaire. Une sonde particulière selon l'invention est représentée par la séquence SEQ ID n° 1 entière ou son brin complémentaire.

De manière plus générale, une sonde de l'invention est composée de tout acide nucléique permettant de détecter la présence du gène de la Marenostrine (i.e., d'hybrider avec tout ou partie du gène de la Marenostrine). Encore plus préférentiellement, une sonde de l'invention est composée d'un acide nucléique permettant de détecter la présence du dernier exon (exon 10) du gène de la Marenostrine. Cet exon est en effet porteur des modifications génétiques décrites ci-avant, responsables de la FMF, et constitue donc une région cible privilégiée pour les expériences de détection. Ainsi, une sonde préférée de l'invention comprend avantageusement tout ou une partie de la séquence comprise entre les résidus 1000 et 1400 de la séquence SEQ ID n° 1 ou de son brin complémentaire. Encore plus particulièrement, la sonde de l'invention recouvre au moins un résidu muté dans le cas de la FMF. Ces résidus sont situés aux positions 1170, 1172, 1267 et 1130 de la séquence SEQ ID n° 1. Des sondes selon l'invention peuvent être spécifiques de chacune (ou plusieurs) de ces mutations et de la séquence non-mutée, et ainsi permettre la détection spécifique des séquences native (non-mutée) ou de l'une ou l'autre des mutations (haplotypes fondateurs).

Ainsi, la présente invention concerne une sonde permettant la détection spécifique de la présence d'une mutation sur le nucléotide 1170 du gène de la marenostrine tel que représenté sur la SEQ ID n° 1. Plus particulièrement, cette mutation est une substitution d'une Adénine par une Guanine. Une telle sonde permet de détecter la présence d'une anomalie génétique caractéristique de l'haplotype fondateur MED (S, ARM1, T, ARA1).

La présente invention concerne également toute sonde permettant la détection spécifique de la présence d'une mutation sur le nucléotide 1172 du gène de la marenostrine tel que représenté sur la SEQ ID n° 1. Plus particulièrement, cette mutation est une substitution d'une Guanine par une Adénine. Une telle sonde permet de détecter la présence d'une anomalie génétique caractéristique de l'haplotype fondateur ARA2.

La présente invention concerne aussi toute sonde permettant la détection spécifique de la présence d'une mutation sur le nucléotide 1267 du gène de la marenostrine tel que représenté sur la SEQ ID n° 1. Plus particulièrement, cette mutation est une substitution d'une Thymine par une Cytosine. Une telle sonde permet de détecter la présence d'une anomalie génétique caractéristique de l'haplotype fondateur ARM3 (ou D).

La présente invention concerne encore toute sonde permettant la détection spécifique de la présence d'une mutation sur le nucléotide 1130 du gène de la marenostrine tel que représenté sur la SEQ ID n° 1. Plus particulièrement, cette mutation est une substitution d'une Guanine par une Cytosine. Une telle sonde permet de détecter la présence d'une anomalie génétique caractéristique de l'haplotype fondateur ARM2.

D'autres sondes selon l'invention sont des sondes permettant la détection de toute autre mutation présente sur l'exon 10 du gène de la marenostrine, telle que par exemple une mutation AAG->AGG (Lys->Arg) sur le nucléotide 1174 ou une délétion de AAT (position 1166) dans les codons ATA.ATG du gène de la marenostrine tel que représenté sur la SEQ ID n° 1.

La présente invention concerne aussi toute sonde permettant la détection spécifique de l'absence de mutation sur les positions 1170, 1172, 1267 et 1130 du gène de la marenostrine tel que représenté sur la SEQ ID n° 1.

La présente invention concerne aussi toute sonde permettant la détection de la présence de l'exon 2 du gène de la marenostrine. Il s'agit en particulier de tout acide nucléique hybridant spécifiquement avec tout ou une partie de l'exon 2, notamment avec une partie au moins de l'exon, portant une mutation observée dans la maladie FMF (mutation E148Q par exemple).

Des sondes de l'invention peuvent également être construites de manière à detecter la présence de plusieurs mutations simultanément, ce qui permet de réduire le nombre d'expérimentations.

Les sondes selon l'invention peuvent par ailleurs être marquées, pour permettre leur détection. Le marquage utilisé peut être tout type de marquage connu de l'homme du métier, permettant de générer un signal. Il peut s'agir d'un marquage radioactif, enzymatique, chimique, immunologique, etc. Ces marquages sont par exemple un marquage au phosphore 32, par la biotine (16-dUTP), par la digoxygénine (11-dUTP), etc.

Un autre objet de l'invention concerne une amorce pour l'amplification d'un acide nucléique tel que défini ci-dessus. En particulier, l'invention concerne toute amorce utilisable pour l'amplification spécifique du gène responsable de la FMF. Plus particulièrement, l'invention concerne toute amorce utilisable pour l'amplification de tout ou une partie du gène dé la Marenostrine.

Le terme amorce désigne communément un acide nucléique simple-brin servant de point de départ pour une réaction d'amplification d'acides nucléiques.

Avantageusement, les amorces de l'invention permettent l'amplification du gène de la Marenostrine. Les amorces de l'invention sont avantageusement composées de de 3 à 50 bases, de préférence de 3 à 40 et encore plus préférentiellement de 3 à 35. Les amorces de l'invention peuvent notamment être issues d'un acide nucléique tel que défini ci-avant, ou d'un acide nucléique dont la carte est représentée sur la figure 3.

Avantageusement, une amorce selon la présente invention est un acide nucléique simple-brin comprenant de 3 à 50 nucléotides complémentaires d'une partie au moins du gène de la marenostrine. Plus particulièrement, une amorce préférée de l'invention est un acide nucléique simple-brin comprenant de 3 à 50 nucléotides complémentaires d'une partie au moins de la séquence ID n° 1 ou de son brin complémentaire.

Selon un mode de réalisation particulier, l'invention concerne des amorces telles que définies ci-avant qui sont complémentaires d'une région au moins du gène de la marenostrine portant une mutation impliquée dans la FMF. Plus particulièrement, des amorces avantageuses de l'invention sont complémentaires d'une partie au moins de la séquence SEQ ID 1 suivantes:
- A -> G qui remplace le codon méthionine ATG en position 1170 sur la séquence SEQ ID n° 1 en un codon valine GTG (variation Med);
- G -> A qui remplace le codon méthionine (ATG) en position 1172 sur la séquence SEQ ID n° 1 en un codon isoleucine (ATA) (variation Ara2);
- T -> C qui remplace le codon valine (GTT) en position 1267 sur la séquence SEQ ID n° 1 en codon alanine (GCT) (variation Arm3);
G -> C qui remplace un le codon méthionine (ATG) en position 1130 sur la séquence SEQ ID n° 1 en un codon isoleucine (ATC) (variation Arm2).

Ainsi, la présente invention concerne plus particulièrement une amorce caractérisée en ce qu'elle comprend de 3 à 50 nucléotides et en ce qu'elle est complémentaire d'une région du gène de la Marenostrine portant une mutation sur le nucléotide 1170 tel que représenté sur la SEQ ID n° 1. Plus particulièrement, cette mutation est une substitution d'une Adénine par une Guanine. Une telle amorce permet, couplée à une amorce appropriée, d'amplifier et de détecter la présence d'une anomalie génétique caractéristique de l'haplotype fondateur MED (S, ARM1, T, ARA1).

La présente invention concerne également toute amorce caractérisée en ce qu'elle comprend de 3 à 50 nucléotides et en ce qu'elle est complémentaire d'une région du gène de la Marenostrine portant une mutation sur le nucléotide 1172 tel que représenté sur la SEQ ID n° 1. Plus particulièrement, cette mutation est une substitution d'une Guanine par une Adénine. Une telle amorce permet, couplée à une amorce appropriée, d'amplifier et de détecter la présence d'une anomalie génétique caractéristique de l'haplotype fondateur ARA2.

La présente invention concerne aussi toute amorce caractérisée en ce qu'elle comprend de 3 à 50 nucléotides et en ce qu'elle est complémentaire d'une région du gène de la Marenostrine portant une mutation sur le nucléotide 1267 tel que représenté sur la SEQ ID n° 1. Plus particulièrement, cette mutation est une substitution d'une Thymine par une Cytosine. Une telle amorce permet, couplée à une amorce appropriée, d'amplifier et de détecter la présence d'une anomalie génétique caractéristique de l'haplotype fondateur ARM3 (ou D).

La présente invention concerne encore toute amorce caractérisée en ce qu'elle comprend de 3 à 50 nucléotides et en ce qu'elle est complémentaire d'une région du gène de la Marenostrine portant une mutation sur le nucléotide 1130 tel que représenté sur la SEQ ID n° 1. Plus particulièrement, cette mutation est une substitution d'une Guanine par une Cytosine. Une telle amorce permet, couplée à une amorce appropriée, d'amplifier et de détecter la présence d'une anomalie génétique caractéristique de l'haplotype fondateur ARM2.

La présente invention concerne également toute amorce caractérisée en ce qu'elle comprend de 3 à 50 nucléotides et en ce qu'elle est complémentaire d'une région du gène de la Marenostrine comprenant au moins l'un des nucléotides 1130, 1170, 1172 et 1267 tels que représentés sur la SEQ ID n° 1, lesdits nucléotides ne présentant pas de mutation. De telles amorces permettent, couplées à une amorce appropriée, d'amplifier et de détecter l'absence d'anomalie génétique dans l'acide nucléique testé.

L'invention concerne également un couple d'amorce, caractérisé en ce qu'il permet l'amplification d'une région comportant au moins l'une des mutations identifiées ci-dessus, c'est-à-dire incluant au moins un des résidus 1130, 1170, 1172 ou 1267 tels que représentés sur la SEQ ID n° 1. Encore plus préférentiellement, le couple d'amorce selon l'invention permet l'amplification d'une région correspondant aux nucléotides 1000 à 1400 environ de la séquence SEQ ID n°1, encore plus préférentiellement 1100 à 1300 environ.

De manière avantageuse, la région amplifiée (i.e., délimitée) par le couple d'amorce de l'invention comprend moins de 1000 pb, plus préférentiellement moins de 500 pb. Dans un mode de réalisation particulier, la région amplifiée comprend moins de 250 pb.

Un mode particulier de couple d'amorce de l'invention comprend :
b) une amorce hybridant sur l'un des brins du gène de la Marenostrine, dans une région comprise entre les résidus 1200 à 1900 tels que représentés sur la séquence ID n° 1, de préférence entre les résidus 1300 à 1600;
c) une amorce hybridant sur l'autre brin du gène de la Marenostrine, dans une région comprise entre les résidus 300 à 1000 tels que représentés sur la séquence ID n° 1, de préférence entre les résidus 500 à 1000, encore plus préférentiellement 700 à 1000;
d) ces deux amorces permettant l'amplification d'une région de 500 pb au plus comportant tout ou partie de la région comprise entre les résidus 300 et 1900 de la séquence SEQ ID 1.

Dans un mode particulier, l'une des amorces des couples définis ci-dessus est une amorce recouvrant un site de mutation dans le gène de la Marenostrine, tel que défini ci-avant.

Un autre objet de l'invention réside également dans un procédé de détection de la présence d'une mutation dans le gène de la Marenostrine comprenant :
a) la mise à disposition d'un échantillon comprenant un acide nucléique et,
b) la détection de la présence d'une ou plusieurs mutations dans cet acide nucléique par hybridation dans des conditions de stringence avec une sonde selon l'une quelconque des revendications 10 à 14.

Dans ce procédé, la mise à disposition d'un échantillon comprend par exemple le prélèvement d'un échantillon biologique chez un sujet, ou simplement l'utilisation d'un échantillon stocké, de manière à rendre l'acide nucléique codant pour la Marenostrine détectable. En particulier, si cet acide nucléique est contenu dans une cellule, l'étape a) comprendra notamment la rupture de cette cellule pour faciliter la détection de l'acide nucléique.

L'acide nucléique tel que défini dans l'étape a) peut être un ADNg, un ADNc ou un ARNm par exemple. Le plus souvent, il s'agira d'un ADNg contenu dans un échantillon biologique prélevé chez un sujet. Par ailleurs, comme indiqué, cet acide nucléique code tout ou une partie de la Marenostrine. Il est en effet possible d'utiliser soit un acide nucléique complet, afin de pouvoir, le cas échéant, détecter la présence de mutations sur l'ensemble de la séquence, soit un acide nucléique codant une partie seulement de la protéine, ladite partie étant susceptible de porter certaines mutations. Avantageusement, l'acide nucléique code toute la Marenostrine ou une partie comportant au moins une partie du dernier exon (exon 10) ou de l'exon 2. Plus particulièrement, la partie préférée correspond au moins à la région comprise entre les résidus 1130 et 1267 de la séquence SEQ ID n° 1 (acides aminés 376 à 423).

Par ailleurs, pour faciliter l'étape de détection, le procédé de l'invention comporte avantageusement une étape, préalable ou concommittante à l'étape b), d'amplification de l'acide nucléique. Cette amplification peut être réalisée par toutes les techniques connues de l'homme du métier. Il peut s'agir notamment d'une amplification par la technique de PCR, en utilisant par exemple des couples d'amorces tels que définis précédemment, suivie ou non d'une étape de clonage des produits d'amplification dans un vecteur plasmidique.

Dans un mode particulier de mise en oeuvre, l'amplification est réalisée en utilisant un couple d'amorces dont l'une recouvre une des positions mutées identifiées ci-avant dans le gène de la Marenostrine. Ainsi, l'amplification ne se produit que lorsque la mutation spécifique de l'amorce est présente dans l'acide nucléique. De ce fait, l'amplification et la détection sont concommitantes, la présence ou non d'un fragment amplifié traduisant directement la présence ou non d'une mutation correspondante.

Comme indiqué ci-avant, compte tenu de la pénétrance des mutations identifiées, l'invention permet également le dépistage et le diagnostic de la FMF. A cet égard, un autre objet de l'invention concerne un procédé de diagnostic in vitro de la FMF comprenant les étapes suivantes :
a) le prélèvement chez un sujet d'un échantillon biologique comportant un acide nucléique codant tout ou une partie de la Marenostrine,
b) la détection dans cet échantillon de la présence de mutation(s) dans ledit acide nucléique.

L'invention concerne en outre un kit (une trousse) utilisable pour la mise en oeuvre d'un procédé tel que défini ci-avant, ledit kit comportant notamment un couple d'amorce tel que défini ci-avant et les réactifs nécessaires à l'amplification d'un acide nucléique. Le kit de l'invention peut comporter en outre un réactif supplémentaire pour la détection d'une mutation, tel que notamment une sonde selon l'invention. Par ailleurs, un kit de l'invention peut également comprendre un support pour la mise en oeuvre du procédé, par exemple une plaque.

Un autre objet de la présente invention réside dans un polypeptide tel que codé par un acide nucléique défini ci-avant. Le terme "polypeptide" désigne au sens de l'invention toute molécule comprenant un enchaînement de 2 acides aminés au moins. Ce terme inclut en particulier les peptides et protéines.

Avantageusement, il s'agit d'un polypeptide comportant tout ou une partie de la séquence d'acides aminés présentée sur la SEQ ID n° 1 ou d'un variant de celle-ci. Une partie comprend notamment tout fragment de cette protéine, comportant avantageusement plus de trois résidus consécutifs, encore plus préférentiellement plus de 10. De tels fragments peuvent être préparés par les techniques connues de l'homme du métier (clivage enzymatique, synthèse chimique, génie génétique, etc). De tels fragments peuvent être utilisés comme antigènes ou comme compétiteurs, ou pour des expériences de modélisation moléculaire par exemple.

Une partie désigne préférentiellement un fragment comportant plus de 20 acides aminés.

Un variant tel qu'indiqué ci-dessus correspond à tout polypeptide dérivant de la séquence SEQ ID n° 1 par des modifications structurales. Préférentiellement, ces modifications affectent moins de 10% des résidus de la séquence, encore plus préférentiellement moins de 5%. Ces modifications peuvent être de nature génétique, chimique ou enzymatique. Des variants tels que définis ci-dessus sont notamment les polypeptides portant les mutations indiquées dans le Tableau 2.

Un objet particulier de l'invention réside dans une protéine codée par le gène comprenant la séquence SEQ ID n°1.

Sur la base de comparaisons de séquences, il est possible d'attribuer plusieurs propriétés à la Marenostrine. Ainsi, une localisation nucléaire et une activité de liaison à l'ADN ou un role dans la régulation de l'expression de gènes ont été proposés pour les protéines SSA-1, Pwa-33, RPT-1 et RFP, qui contiennent toutes également des motifs N-terminaux en doigt-de-Zinc. Ainsi, la Marenostrine pourrait appartenir à une famille de facteurs de régulation, liant l'ADN, et pourrait ainsi réguler l'expression de gènes, éventuellement de plusieurs gènes. La protéine rpt-1 a notamment été impliquée dans la régulation de l'expression d'une chaine du recepteur de l'interleukine-2. Les membres de cette famille de gènes peuvent donc être impliqués dans la régulation de l'expression de protéine ayant un role dans l'immunité, une observation qui pourrait être reliée à l'inflammation récurrente observée dans la FMF. Par ailleurs, la protéine BT (butyrophyline), qui contient également un domaine de type rfp mais est dépourvue de régions en doigt -de-zinc, est une glycoprotéine transmembranaire exprimée dans les tissus mammaires pendant la phase de lactation.

L'invention concerne en outre l'utilisation thérapeutique des acides nucléiques et polypeptides décrits ci-dessus. En particulier, un acide nucléique codant la Marenostrine humaine, ou la protéine correspondante, peuvent être utilisés pour la mise au point de thérapies par restauration de l'activité déficiente. L'acide nucléique peut notamment être utilisé dans le cadre de thérapies géniques. La protéine recombinante native (i.e. non mutée) peut être préparée par expression, dans un hôte cellulaire, d'un acide nucléique tel que défini dans la présente demande.

A cet égard, l'invention concerne en outre un procédé de production de la Marenostrine recombinante comprenant la culture d'une cellule comprenant un acide nucléique codant la Marenostrine, et la récupération de la protéine ainsi produite. Ladite cellule est par exemple une cellule procaryote (bactérie) ou eucaryote (levure, cellule animale, etc). La cellule est une cellule dans laquelle l'acide nucléique a été introduit artificiellement, ou une cellule descendant d'une telle cellule.

L'invention concerne en outre une composition pharmaceutique comprenant un acide nucléique ou un polypeptide tel que défini ci-avant.

Les acides nucléiques et polypeptides de l'invention peuvent également être utilisés pour la recherche de molécules actives, notamment de molécules capables d'agir sur la FMF. Ainsi, les polypeptides peuvent être utilisés pour générer, par exemple par modélisation moléculaire, des produits non-peptidiques ou non-entièrement peptidiques, possédant la capacité de traiter la FMF.

Les acides nucléiques et polypeptides de l'invention peuvent aussi être utilisés pour générer, selon les méthodes connues de l'homme du métier, des anticorps polyclonaux ou monoclonaux correspondants. De tels anticorps sont par exemple utilisables pour la détection de la présence de Marenostrine dans un échantillon, ou pour la purification de la Marenostrine.

La présente invention sera décrite plus en détails à l'aide des exemples qui suivent, qui doivent être considérés comme illustratifs et non limitatifs.

### Légende des Figures

### Figure 1

Carte génétique et physique de l'intervalle candidat *MEFV.* Les lignes horizontales représentent l'étendue de chaque clone YAC : 16Gh7, 633d12 et 26Fe7 (lignes épaisses) et clones cosmide : BC2, BD6, AA6, 30c3, 30e1, 30g4, 30f9 et 30e10 (lignes minces). Les délétions dans le YAC 633d12 et dans le cosmide AA6 sont représentées par des lignes pointillées. Les lacunes dans les séquences sont représentées par des doubles barres obliques (//).

### Figure 2

Recombinaisons historiques dans l'haplotype fondateur *MED.* Les chromosomes porteurs FMF trouvés dans les sujets Juifs nord-africains 68-3 et 91-3 et dans les sujets Arméniens A26-3 et A28-1 sont représentés. La position physique des marqueurs microsatellite et biallelique est montrée sur le dessus. L'étendue de l'haplotype fondateur Méditerranéen est indiqué par un cadre : comme indiqué antérieurement (pour D16S3275, les deux allèles 9 et 18 ont été observés dans cet haplotype fondateur) les haplotypes Méditerranéen S et ARM1 diffèrent par des marqueurs distaux D13S3082. Le marqueur D16S3373 est au même locus que AFMef43. Pour les marqueurs bialleliques, x/y indique des marqueurs hétérozygotes non-phasés ; les génotypages non-disponibles ont été laissés en blanc. Tel : coté telomerique ; Cen : coté centromerique.

### Figure 3

Carte de transcription de la région FMF. La structure génomique des gènes *Znfmf* et *Marenostrin* est indiquée par des diagrammes montrant les exons (boîtes) et les introns (lignes). Les flèches horizontales indiquent la direction de la transcription. La séquence est présentée avec la direction centromèrique vers la droite. La localisation de séquences EST est indiquée ; lorsque deux EST représentent deux extrémités du même clone, elles sont représentées par des boîtes.

### Figure 4

(A) Séquence SEQ ID n° 1 : Séquence d'ADNc partielle et séquence d'acides aminés déduite de la Marenostrin. La numérotation du clone d'ADNc commence au premier nucléotide (marge droite) et la numérotation de sa traduction en code à une lettre (bas) commence au premier amino acide (marge de gauche).
(B) Comparaison de la traduction conceptuelle aux domaines définis dans ProDom : domaine 4240 (résidus 81 à 138 de la marenostrin), domaine 7680 (139 à 219), domaine 9608 (220 à 263), domaine 7736 (293 à 461). RFP_MOUSE et RFP_HUMAN : protéines de doigt ret de souris et humaine, RO52_HUMAN : antigène du syndrome Sjogren de type A. RPT : protéine régulatrice, lymphocyte T, 1, A33_PLEWA : protéine en doigt de zinc liant l'ADN de pleurodeles waltii, BUTY_MOUSE, BUTY_HUMAN, BUTY_BOVIN : Butyrophilin de souris, humaine et de vache. Les points indiquent les espacements de manière à optimiser l'alignement des séquences.

### Figure 5

Haplotypes fondateurs FMF observés dans différentes populations affectées. Les haplotypes sont groupés pour schématiser le segment de chromosomes retenus de chacun des quatre haplotypes fondateurs associés avec des variations de séquences de la Marenostrin. (N), nombre d'haplotype portant la combinaison allelique encadrée. Origine ethnique : A : Arméniens ; NAJ : Juifs Non-Ashkenazes (incluant des Juifs d'Afrique du Nord et d'Iraq) ; D : Druzes ; T : Turques ; MA : Arabes du Maghreb ; O : autres.

### Matériels et Méthodes

### 1. Construction des cosmides portant les contig.

Les YACs 16Gh7, 633d12 et 26Fe7 ont été digérés partiellement avec l'enzyme Sau3A, déphosphorylés, et ligaturés dans le site BamHI du vecteur cosmide sCOGH2 ou RH3. Ces vecteurs ont ensuite été utilisés pour transfecter la souche E. Coli STBL2 (selon les protocoles standards). Les cosmides issus de clones hybridant à l'ADN humain, ont été purifiés et étalés au moyen d'un robot Hybaid sur des membranes de nylon (Hybond N+). Les filtres ont ensuite été hybridés avec des sondes non radioactives synthétisées à partir d'amorces STS et des extrémités du cosmide.

Les amorces ont été marquées avec de la biotine-16-dd-UTP (Boehringer Mannheim) en utilisant la terminale transferase, puis hybridées au filtre selon des protocoles standards. Des sondes d'extrémités des clones ont ensuite été générées par PCR asymétrique en utilisant une amorce vecteur (hGHlink.up: ACAATGGGAGCTGGTCTC (SEQ ID NO:2) ou hGHlink.lo: TAGGAGAAGGACCGCCCA (SEQ ID NO:3)) dans un volume réactionnel de 50 µl en présence de 500 ng d'ADN du cosmide, 10 µM de digoxygenine-11-dUTP (Boehringer Mannheim), 90 µM de dDTP, 100 µM de chaque d(ACG)TP, 0,5 µM d'amorce, 100 µM de Tris-HCI, pH8,8, 50 mM de Kcl, 1,5 mM de MgCl₂ et 0,1 % de Triton X-100. Une unité d'ADN polymérase Taq (Cetus) a été ajoutée après 5 mn à 90°C et le mélange a été soumis à 30 cycles d'amplification, comme suit : 25 secondes à 94°C, 30 secondes à 60°C, et 1 minute à 72°C. Les sondes ont été vérifiées sur un gel d'agarose à 0,8 % et purifiées sur une colonne G25 Sephadex. Les hybridations sur filtre ont été réalisées dans un tampon DIG Easy Hyb (Boehringer Mannheim), en présence d'un ADN compétiteur (cot1 DNA, Boehringer Mannheim). Pour la détection du signal, le substrat CDP-StarTM de la phosphatase alcaline a été utilisé (Boehringer Mannheim). Les analyses de données et l'assemblage des contigs ont été réalisées en utilisant le Contigs Package (ref. 26).

### 2. Séquençage par "Pairwise end sequencing"

Pour chaque cosmide, les fragments d'une digestion partielle par l'enzyme CviJI ont été purifiés sur gel pour une taille de 8 à 10 kb, sous-clonés dans le plasmide pBC-SK+ (CmR, Stratagene) et sequencés sur les deux extrémités. L'assemblage d'une moyenne de 300 lectures exploitables, en utilisant les logiciels PHRED et PHRAP (P. Green, communication personnelle), a résulté en un assemblage de sous-clones pour chaque cosmide permettant de positionner les séquences des contigs (en terme de distance et d'orientation relatives ainsi que les lacunes résultantes (en position et en taille) permettant ainsi de diriger les opérations terminales par marche au moyen d'une amorce par exemple. Cette procédure s'est avérée très efficace pour la résolution de région hautement répétitive ou de duplication pratiquement exacte (ref. 8).

### 3. Validation des contigs et des séquences

Le caractère chimérique des YACs a été contrôlé par hybridation fluorescente *in situ* (FISH) sur des métaphases étalées de donneurs sains en utilisant l'ADN total de clones de YACs ou l'inter-Alu PCR en tant que sonde (ref. 27). La cartographie par Fiber FISH a été réalisée par co-hybridation de certains cosmides sélectionnés comme sondes (marqués par la biotine 16-dUTP et par la digoxygenine 11-dUTP) sur l'ADN étendu préparé (i) à partir de lignées cellulaires lymphoblastoides de donneurs sains, (ii) à partir du YAC 26Fe7 non chimérique en tant que référence et (iii) à partir du clone YAC délété 633d12. Les sondes marquées à la biotine et la digoxygenine ont été détectées par Texas Red et FITC respectivement et visualisées simultanément avec un filtre à double bande en utilisant le microscope à epifluorescence Leica DMRB. Les images ont été enregistrées avec une caméra CCD. Des cycles successifs de co-hybridations de trois cosmides ont permis de couvrir la délétion du YAC 633d12.

La fidélité à la séquence humaine a été vérifiée (i) en comparant la carte de restriction déduite de la séquence (pour EcoRI, Bglll, NotI, Eagl, Mlul et BssHII) au profil de restriction observé sur tous les cosmides assemblés dans la région d'intérêt ou aux données publiées ; (ii) par analyse PCR Long Range systématique d'ADN contrôle ; (iii) en certaines situations critiques, par analyse Southern-blot, en utilisant l'ADN digéré d'échantillons contrôle. De plus, pour tous les exons détectés, plus de la moitié de la séquence de la région critique (plus de 35 kb au total) ont été re-séquencés à partir d'ADN "normal" et "affecté".

### 4. Mise au point de marqueurs des séquences bi-alléliques

Sur la base de données de séquençage, 8 fragments PCR (3 à 8 kb de longueur) ont été générés dans l'intervalle *MEFV.* La technique de PCR-LA (Takara) a été utilisée pour préparer des fragments d'ADN correspondants à partir de patient et d'individus sains de familles FMF. Ces matrices PCR ont ensuite été purifiées en utilisant le système Microcon (Amicon) et soumises à une étape de séquençage cyclique par terminateurs fluorescents. Les comparaisons de séquences ont été effectuées en utilisant le système Staden96. Les amorces ont été générées avec le logiciel Oligo 4.0 (ref. 30). Des informations précises sur les nouveaux marqueurs sont disponibles à partir du Génome Data Base (GDB).

### 5. Marqueurs génétiques

Des informations sur les microsatellites marqueurs ont été obtenues à partir de la Banque Genome Data Base (GDB). Les marqueurs AFMef51 et AFMef52 ont été décrits antérieurement (The French FMF Consortium, submitted).

### 6. Prédiction de gènes

Les programmes BLAST ont été utilisés pour déterminer les ressemblances et les identités avec des gènes connus en utilisant une compilation non-redondante des bases EMBL et GenBank. Les comparaisons d'acides Aminés ont été réalisées en traduisant les séquences d'ADN dans les six cadres de lecture potentiels et en comparant ces traductions à des séquences de protéines trouvées dans des Bases de Données Swiss-Prot et PIR en utilisant le programme BLASTX. Les domaines d'homologies de protéine ont été identifiés par recherche dans la banque de protéines PROSITE.

Les prédictions d'exon ont été réalisées en utilisant plusieurs programmes : le programme GRAIL, le programme FGENEH et le programme GENIE. L'accès à ces programmes a été réalisé sur le serveur électronique.

### 7. Exon-Trapping

L'ADN cosmidique a été partiellement digéré par l'enzyme Sau3A et ligaturé au vecteur pSPL3 (BRL). Le produit de ligation a été transféré dans la cellule hôte *E. coli XL1-blue* par electroporation. L'ADN plasmidique a été récolté et introduit dans les cellules COS-7 par electroporation. L'ARN a été isolé 48 heures après la transfection et converti en ADNc simple-brin en présence de transcriptase inverse SuperScript II (BRL). L'ADNc simple-brin a ensuite été transformé en ADN double-brin par six cycles d'amplification PCR. Pour éliminer les produits d'amplification du vecteur seul et les faux-positifs, l'enzyme BstXI a été ajoutée à la réaction et incubée pour la nuit. Les produits ARN de la PCR ont ensuite été clonés dans le vecteur pAMP (BRL), et des clones individuels ont été séquencés.

### 8. Amplification d'ADNc

Des fragments d'ADNc correspondants aux gènes de la *Marenostrin* et *Znfmf* ont été amplifiés à partir de banques d'ADNc ou d'ADNc Quick clone (Clontech). Des amorces ont été générées à partir des séquences de produits de l'Exon-trapping et utilisés dans des réactions de PCR standards. Des analyses par RACE ont été réalisées en 3' et en 5' pour caractériser les extrémités de ces gènes en utilisant le kit d'amplification d'ADNc marathon (Clontech) selon les recommandations du fabriquant. Des réactions de PCR ont ensuite été réalisées en utilisant des oligonucléotides de l'adaptateur (AP1 et AP2) ainsi que des oligonucléotides internes aux séquences d'ADNc. Les produits de ces PCR ont été clonés dans le vecteur TA (Invitrogen) et des clones recombinants ont été séquencés.

### 9. Identification de mutations

Les exons et les introns flanquants ont été amplifiés par PCR en utilisant des amorces spécifiques suivantes :
Couple 1: 5'-GGAGCACCTGAGAGTGCCACCCACC-3' (SEQ ID NO:4) 5'-ATTGGCGCTCAGGCACAT-3' (SEQ ID NO:5)
Couple 2: 5'-CGGAGAGCCCAGAACAATGATACAGC-3' (SEQ ID NO:6) 5'-GCCAAAGTTGAGGACCAGCATTTAGACC-3' (SEQ ID NO:7)
Couple 3: 5'-GCCCTTCTCCCTATCAAATCC-3' (SEQ ID NO:8) 5'-CCACCTCTTATCCACCTCC-3' (SEQ ID NO:9)
Couple 4:

5'-CACAGCAGAATCTCGGGG-3' (SEQ ID NO:10)

5'-GCACATCTCAGGCAAGGG-3' (SEQ ID NO:11)

Les conditions suivantes ont été utilisées pour la PCR : dénaturation à 95°C pendant 10 mn suivis de 30 cycles de dénaturation à 95°C pendant 15 s, annealing à 55°C pendant 30 s, puis élongation à 72°C pendant 3 mn et élongation finale à 72 °C pendant 10 mn. Les produits de la PCR ont été purifiés par chromatographie sur Sephadex P100 et directement séquencés en utilisant des amorces spécifiques et le kit de séquençage cyclique AmpliTaq FS Dye Terminator au moyen du séquenceur ABI prism 377.

### 10. Criblage des mutations

a) Variants *Arm2* et *Arm3*
   L'exon situé le plus en 3' a été amplifié à partir d'ADN génomique en utilisant les amorces p12.2 (TATCATTGTTCTGGGCTC, SEQ ID NO:12) et p10.1 (CTCCGTACTTCCTCCTCT, SEQ ID NO:13). Les produits de la PCR ont été digérés selon les recommandations du fabriquant par les enzymes Alul et Hinfl pour le criblage de mutation T/C (1267) et G/C (1130) respectivement. Dans le premier cas, un site Alul est créé et dans le second cas, un site Hinfi est détruit. Les produits de la digestion ont été analysés sur gels de polycryamide de 6%.
b) Variants *Med* et *Ara2*
   Les mutations A/G (1170) et G/A (1172) ne modifient aucun site de restriction connu. De ce fait, des amorces allele-specifiques ont été créées. La mutation Med a été distinguée en utilisant les oligonucléotides Met1 (TGGTACTCATTTTCCTCCAT, SEQ ID NO:14) et p12.2 (voir ci-dessus) et avec les oligonucléotides Val1 (TGGTACTCATTTTCCTTCAC, SEQ ID NO:15) et p12.2. La mutation Ara2 a été contrôlée avec les oligonucléotides met2 (CTGGTACTCATTTTCCTTC, SEQ ID NO:16) et p12.2 ainsi que les oligonucléotides ilel (CTGGTACTCATTTTCCTTT, SEQ ID NO:17) et p122. Les produits de PCR ont été analysés sur gels Nusieve 3% / Seakem 1%.

### 11. Analyse en Northern Blot

Les Northern Blot (Clontech) ont été pré-hybridés dans un tampon ExpressHyb (Clontech) et hybridés dans la même solution contenant la sonde ADNC marquée au phosphore ³². Les membranes ont été lavées à 50°C avec 0.1x de solution saline citrate standard (SSC) et 0.1 % de SDS, puis exposés sur des films sensibles aux rayons X à - 80°C pendant 7 jours.

### Exemples

### 1. Séquençage de l'intervalle compris entre les marqueurs D16S3070 et D16S3275

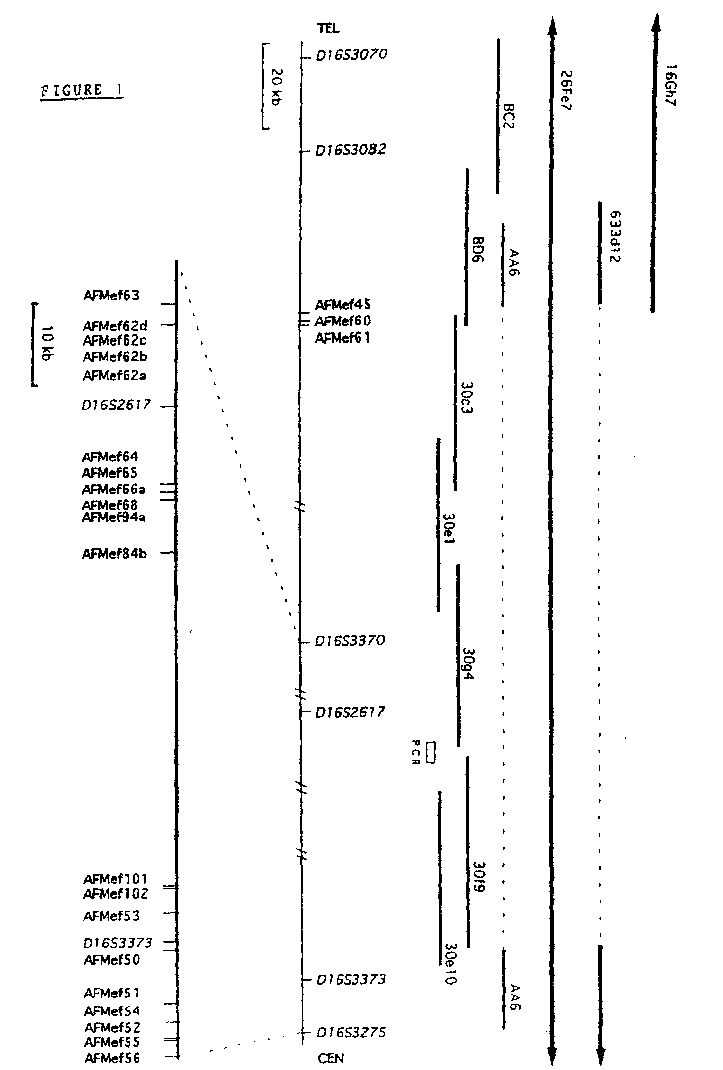

Dans le but d'analyser de manière exhaustive l'existence de gènes et la présence de polymorphismes dans l'intervalle ci-dessus, le séquençage complet de cette région à été réalisé. Dans ce but, une carte de préparation au séquençage a été établie dans un premier temps comme suit : L'intervalle candidat a été sous-cloné à partir des YACs 16Gh7 et 633d12 (isolés à partir des banques ICI et CEPH respectivement) dans le vecteur cosmide sCOGH2 (ref. 7). L'assemblage des cosmides en contig a été effectué sur la base du contenu en STS, à partir de marqueurs génétiques et de sondes d'extrémités de YACs et de cosmides. L'assemblage correspondant au contig minimum est représenté sur la Figure 1. La continuité du contig a été controlée par la technique de FISH sur des chromosomes métaphasiques et sur des fibres étendues d'ADN génomiques. Cette analyse a mis en évidence la présence d'une délétion de 160 kD environ dans le YAC 633d12 (Figure 1). Cette partie de la séquence a pu être obtenue par des méthodes complémentaires, et notamment à partir de sous-clones de cosmides dérivés du clone YAC 26Fe7 de 250 kb (provenant de la banque ICI), qui contient les marqueurs D16S3070 et D16S3275. En tout, 8 cosmides ont du être utilisés pour couvrir la totalité de la région, à l'exception d'un segment supplémentaire de 3 Kb, qui a été cloné à partir d'un produit d'amplification par PCR d'ADN génomique. La taille totale de l'intervalle candidat ainsi cartographié a été estimée à 250 kb, entre les marqueurs D16S3070 et D16S3275. Le séquençage a été réalisé selon la technique de "pairwise end sequencing" (Cf matériels et méthodes).

Une séquence totale de 239 kb a ainsi été assemblée en 5 contigs, séparés par 4 gaps de séquençage (de 200 à 650 pb), qui étaient réfractaires aux techniques de séquençage utilisées. Après avoir vérifié la fidélité de cette séquence à l'ADN humain, cette séquence a été utilisée pour une étude plus fine de l'intervalle et pour définir les unités transcriptionnelles contenues dans celui-ci.

### 2. Etude fine de la séquence de l'intervalle

Lors d'une analyse des recombinaisons dans un haplotype fondateur juif, une région candidate avait été préalablement identifiée dans un intervalle de 250 kb comprise entre les marqueurs D16S3070 et D16S3275 (ref. 5 et 6). La demanderesse a montré plus récemment l'existence d'haplotype fondateur commun entre les populations juives d'Afrique du nord, Arméniennes, Turques et Arabes du Maghreb. L'étude de cet haplotype fondateur commun (MED) dans notre panel étendu et multi-ethnique de patients a suggéré un affinement de cette région candidate à un intervalle compris entre les marqueurs D16S2617 et D16S3373. Une recherche de variations de séquences dans cet intervalle a donc été entreprise pour tenter de confirmer l'analyse d'haplotypes initiale et tenter de localiser de manière plus précise les événements potentiels de recombinaisons qui se produisent dans l'haplotype fondateur MED.

Les analyses de séquences réalisées sur l'intervalle entier ont permis de montrer qu'il n'y avait pas d'autres microsatellites présents dans cette région, susceptibles d'être utilisés comme marqueurs génétiques.

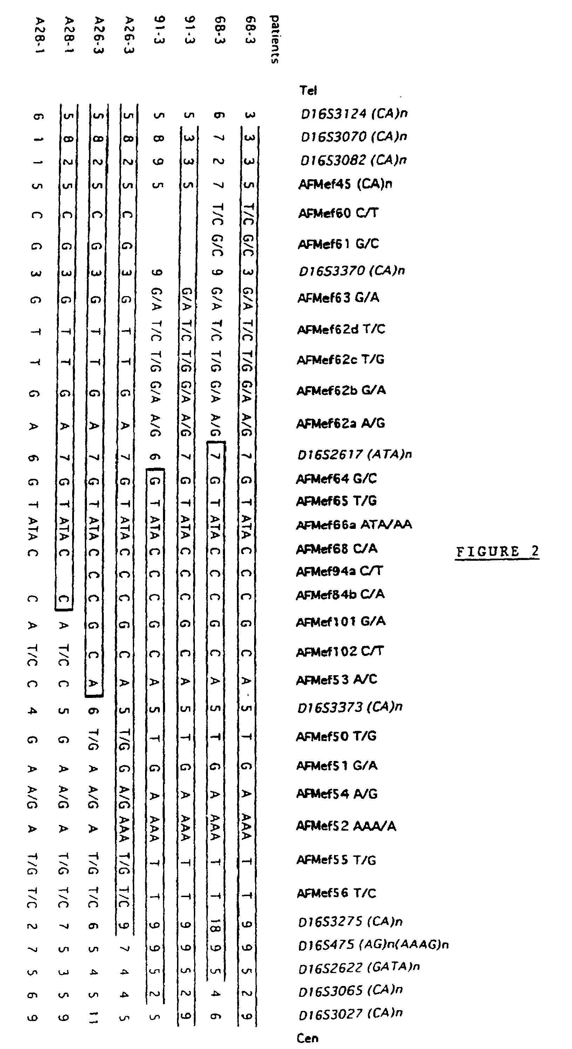

Pour tenter d'analyser cette région, la demanderesse a donc mis en oeuvre une technique différente, consistant à rechercher la présence de polymorphismes bialléliques. A cet effet, plusieurs fragments de 3 à 8 kb ont été séquencés et comparés chez des patients et chez des individus non-porteurs de familles FMF. Cette étude a permis de mettre en évidence et cartographier 22 variants de séquences (Figure 1). Ces 22 variants ont été utilisés comme marqueurs et testés sur différentes familles FMF, et ont permis de révéler des allèles en déséquilibre de liaison avec la maladie, en accord avec l'haplotype fondateur commun MED déduit des analyses de microsatellites. Cette analyse a permis d'affiner la localisation du gène candidat, par déductions à partir d'analyses d'haplotypes fondateurs tronqués par recombinaisons chez des sujets juifs (91-3 et 68-3) et arméniens (A26-3 et A28-1) (voir figure 2). Les patients 91-3 et 68-3 présentent une copie de l'haplotype fondateur MED couvrant la région complète et une deuxième copie tronquée qui recouvre uniquement la partie proximate de cet haplotype, jusqu'au marqueur D16S2617. Alors que les variants bialléliques de la partie centromérique sont homozygotes chez les deux patients, les variants de séquence dans la partie distale du marqueur D16S2617 sont hétérozygotes, révélant la présence d'une région distincte de l'haplotype fondateur dans ce segment. Cette étude a permis de conclure que les patients 91-3 et 68-3 présentent une recombinaison ancéstrale permettant l'exclusion de la région distale au marqueur D16S2617.

De plus, le patient A26-3 est homozygote pour l'haplotype MED dans la portion distale du marqueur D16S3373. De manière similaire aux patients 68-3 et 91-3, des variants hétérozygotes observés pour les marqueurs proximaux indiquent la localisation d'au moins une recombinaison ancestrale excluant la région centromérique au marqueur D16S3373. La phase de D16S3373, afmef51, afmef52 et D16S3275 a pu être déterminée chez le patient A26-3 et indique que les allèles fondateurs (5, G, AAA et 9 respectivement) sont sur le même chromosome, qui porte donc l'haplotype fondateur étendu jusqu'au marqueur D16S3275. Le patient A28-1 présente seulement une copie de l'haplotype MED couvrant les marqueurs D16S3124 à D16S2617, et potentiellement D16S3373. Cependant, ce dernier marqueur ne présente pas de déséquilibre de liaison significatif. Dans la mesure où les marqueurs afmef101 et afmef53 sont homozygotes pour l'allèle non-fondateur (A/A et C/C respectivement) chez ce patient, ces marqueurs peuvent être exclus de l'haplotype fondateur MED porté par ce chromosome. En conséquence, le point de cassure ancestral défini dans A28-1 semble être localisé du coté distal de afmef101, ce qui rapproche la limite centromérique de quelques kb supplémentaires, du marqueur D16S3373 à afmef101. Ceci nous a permis de conclure que la région candidate est bordée par D16S2617 et afmef101- D16S3373 et, selon la carte de la séquence, recouvre un intervalle de 60 kb (Figures 1 et 3).

### 3. Analyse de séquence de l'intervalle de 60 kb

Des exons putatifs ont été identifiés dans les séquences génomiques recouvrant l'intervalle candidat en utilisant plusieurs programmes informatiques. La séquence a tout d'abord été comparée à des banques de données publiques (ref. 9). Des comparaisons avec des banques de EST ("expressed Sequence Tag") (ref. 10, 11) ont ensuite permis de détecter des complémentarités parfaites avec 16 EST humains (Figure 3).

La séquence complète a également été analysée par trois programmes de prédiction d'exons : GRAIL (ref 12), FGENEH (ref 13) et GENIE (ref 14) (Cf matériels et méthodes). Un grand nombre de séquences ont été classées pour leur probabilité élevée d'être des exons. Les résultats obtenus sont présentés dans le tableau 3 ci-dessous :

**Tableau 3**

| | Prédictions | Confirmations |
|---|---|---|
| GRAIL | 34 (22) | 12 (11) |
| BCM | 63 (38) | 12 (11) |
| GENIE | 64 (37) | 13 (12) |
| Recouvrements | 10 (9) | 9 (8) |

Les nombres entre parenthèses représentent les exons putatifs localisés entre les marqueurs D16S2617 et D16S3373. La ligne "Recouvrements" indique les régions identifiées comme exons putatifs par les 3 programmes.

Ces prédictions ont ensuite été testées selon les techniques indiquées dans les matériels et méthodes et se sont montrées dans certains cas déceptives : ainsi, une transcription n'a été détectée que pour 20 à 35% de ces séquences. Cependant, les régions identifiées comme exons putatifs avec les trois programmes se sont révélées être de vrais exons dans presque tous les cas.

Ces prédictions d'exons ainsi que des etudes de comparaisons avec des banques d'acides nucléiques et de protéines ont permis de détecter quatre régions homologues à des gènes identifiés :
- Les deux régions les plus distales présentent une homologie importante avec des gènes codant pour des récepteurs olfactifs (ref 15), qui sont généralement codés par un seul exon.
- Une troisième région observée (désignée Znfmf) présente des homologies au niveau nucléique et protéique avec des région codant pour des motifs doigt-de-zinc d'un grand nombre d'organismes.
- Une quatrième région identifiée (désignée Marenostrine) présente des homologies avec plusieurs protéines contenant un domaine de type rfp.

Dans la mesure où plusieurs de ces exons putatifs et régions d'homologies font probablement partie d'unités transcriptionnelles plus grandes, des expériences supplémentaires de validations et de recherche ont été entreprises par RT-PCR, PCR sur banques d'ADNc et RACE-PCR.

### 4. Exon-Trapping

Des analyses par exon-trapping ("piégeage d'exon") ont été réalisées sur les cosmides 30f9, 30g4 et 30e10, selon le protocole décrit dans les matériels et méthodes. Ces analyses ont conduit à l'identification de 32 exons putatifs, dont 19 localisés entre les marqueurs D16S2617 et D16S3373. La comparaison de la séquence de ces exons avec de l'ADN génomique a permis d'aligner et d'orienter ces fragments par rapport aux clones génomiques. Les séquences identifiées par Exon-Trapping ont également été comparées aux banques d'EST et aux exons identifiés en 3. ci-dessus. Toutes ces données ont ensuite été intégrées pour reconstituer les unités transcriptionnelles. Ces Unités sont représentées sur la Figure 3.

### 5. Isolement d'ADN complémentaires

Dans le but de confirmer la transcription effective de ces exons et unités transcriptionnelles putatifs, des expériences de PCR ont été réalisées. Dans ce but, des produits d'amplification par PCR à partir de banques d'ADNc humain (Clontech), de taille compatible avec les prédictions, ont été séquencés. Les ADNc des deux gènes multiexoniques identifiés en 3. ci-avant, désignés Znfmf et Marenostrine, ont ensuite été étendus en utilisant la technique de RACE-PCR. La position des sites d'épissage de ces gènes a ensuite été déduite par alignement des exons putatifs avec la séquence génomique, tous ces sites présentant bona fide des motifs 5' et 3' consensus.

Des fragments d'ADNc correspondant au gène Znfmf ont ensuite été amplifiés à partir d'une banque d'ADNc de foie, et assemblés en un transcript de 3060 nucléotides de longueur. Ce gène est composé de 5 exons, distribués sur 14 kb d'ADN génomique. Plusieurs ESTs sont inclus dans l'exon le plus en 3' du gène Znfmf, et la séquence AA375800 couvre le deuxième exon de ce gène (Figure 3).

Une séquence d'ADNc de 1,9 kb du gène de la Marenostrine a été obtenue à partir de 8 séquences recouvrantes amplifiées à partir d'une banque d'ADNc de leukocytes. Le gène de la Marenostrine comporte au moins 10 exons, répartis sur plus de 10 kb d'ADN génomique (Figure 3). Aucune complémentarité avec des EST n'a été trouvée dans les banques de séquences publiques.

Afin de rechercher l'ARN de la Marenostrine humaine, une sonde d'ADNc correspondant au gène de la Marenostrine a été hybridée avec un Northern blot d'ARNs poly(A) isolés à partir de différents tissus humains.

Les résultats obtenus font apparaitre une bande faible de 4kb environ, dans les ARNm de leucocytes sanguins périphériques. En revanche, dans les conditions testées, aucune expression n'a été détectée dans les autres tissus testés : rate, thymus, prostate, testicule, utérus, intestin grêle, colon, estomac, thyroïde, moelle épinière, ganglion lymphatique, trachée, glandes surrénales, coeur, cerveau, placenta, poumon, muscle, rein et pancréas. Toutefois, une expression du gène a été détectée par PCR dans des banques d'ADNc de rate et de leucocytes, et par RT-PCR dans la synovie. Les produits de PCR observés dans ces tissus avaient la taille attendue et leur séquence est identique à celle de fragments du gène de la Marenostrine.

### 6. Analyse de la séquence codante

La séquence des 4 régions codantes identifiées dans l'intervalle candidat (Cf 3. ci-dessus) a été analysée afin de déterminer la structure et l'organisation de ces régions ainsi que la nature des protéines correspondantes.
- Parmi les deux régions homologues à des gènes de récepteurs olfactifs, l'une contient une phase de lecture ouverte (ORF) ininterrompue, qui correspond très probablement à un gène fonctionnel. Ce gène putatif a été désigné Olfmf. La traduction conceptuelle de la seconde région homologue à cette superfamille ne contient pas de méthionine à son extrémité amino-terminale, et représente donc vraisemblablement un pseudo-gène (voir Figure 3).
- Le gène Znfmf contient une seule phase ouverte de lecture de 394 acides aminés. La séquence d'acides aminés correspond à un polypeptyide dépourvu de domaine transmembranaire apparent. Le dernier exon code pour 5 domaines en doigt-de-zinc contigus : les deux premiers correspondent parfaitement à la séquence consensus du groupe C2H2 (Y/F-X-C-X2-4-C-X3-F-X5-L-X2-H-X3-4-H-X6-7), et la liaison H-C qui suit chacun d'entre-eux est aussi strictement conservée (TGE-R/K-P-F/Y-X). Ces deux domaines sont suivis par trois autres domaines en doigt-de-zinc pour lesquels la structure et les jonctions sont moins conservées. Le gène Znfmf présente des homologies avec de nombreux autres gènes contenant des domaines en doigt-de-zinc.
- L'ADNc codant la Marenostrine contient une phase de lecture ouverte unique de 477 acides aminés, qui s'étend du nucléotide 1 à 1433 sur les Figures 3 et 4A. Cette séquence ne contient pas le codon méthionine initiateur, ce qui indique qu'une partie 5' de l'ARNm correspondant manque à cette séquence.
   . L'extrémité carboxylique de cette chaine polypeptidique (depuis le résidu 83 jusqu'à la fin) présente des similitudes significatives avec le domaine rfp, dont le prototype a été décrit pour la première fois dans la protéine RFP ("RET Finger Protein", ref. 17). Ce domaine a depuis été retrouvé dans un certain nombre d'autres protéines, et notamment dans le précurseur de la Butyrophyline (BT) ; dans une protéine en doigt-de-zinc liant l'ADN de l'amphibien Pleurodeles waltii (Pwa33) ; dans la protéine RPT-1 de souris et dans l'antigène de type A du syndrome Sjorgen (SS-A). Le domaine de type RFP est représenté en PRODOM par 4 domaines (4240, 7680, 9608, 7736). Aucune structure tridimensionnelle n'est disponible pour ces domaines.
   . La partie amino-terminale de cette chaîne (résidus 1-82) est une région riche en Cys/His, qui pourrait être impliquée dans la liaison de métaux.

### 7. Etudes des variations de séquences chez les patients FMF

Une première étude a été réalisée par la technique de "long range PCR", afin de rechercher l'existence de réarrangements importants dans l'ADN génomique de patients atteints de FMF. Aucun réarrangement de ce type n'a pu être mis en évidence.

Dans un deuxième temps, les exons codant des gènes Olfmf, Znfmf et Marenostrine ont été séquencés chez 7 patients atteints de FMF, qui étaient homozygotes pour un des haplotypes fondateurs, et chez 11 sujets contrôles non-affectés. Le séquençage a été réalisé sur des produits d'amplification d'ADN génomique, pour pouvoir analyser la région codante entière de ces gènes.

Les résultats obtenus montrent que le récepteur olfactif présente une substitution non-conservative chez les individus arabes testés (T/C -> Phe/Ser). Néanmoins, cette substitution a été retrouvée également chez les sujets contrôles de différents groupes ethniques.

Les résultats montrent également l'absence de toute variation de séquence dans les exons codant du gène Znfmf.

En revanche, 4 altérations de séquence différentes, introduisant des changements dans le produit d'expression (protéine) ont été identifiées dans la séquence du gène de la Marenostrine dans différents groupes ethniques. Plus particulièrement, ces 4 altérations de séquences sont localisées dans l'exon le plus en 3' du gène, tel que représenté dans le Tableau 2 ci-dessous :

**Tableau 2**

| Haplotype | Changement de nucléotide | Position (SEQ ID n° 1) | Effet sur le codon |
|---|---|---|---|
| MED | ATG -> GTG | 1170 | Met -> Val |
| ARA2 | ATG -> ATA | 1172 | Met -> Ile |
| D | GTT -> GCT | 1267 | Val -> Ala |
| ARM3 | GTT -> GCT | 1267 | Val -> Ala |
| ARM2 | ATG -> ATC | 1130 | Met -> Ile |

La première altération de séquence (variation Med) consiste en un changement A -> G qui remplace un codon méthionine (ATG) en codon valine (GTG). Cette mutation a été retrouvée dans des familles d'origine ethnique différente (Juives, Arméniennes, Turques et Arabes) présentant l'haplotype MED. Cette altération est donc caractéristique de l'haplotype fondateur MED.

La deuxième altération de séquence (variation Ara2) affecte le même codon que la première et consiste en un changement G -> A qui remplace le même codon méthionine (ATG) en codon isoleucine (ATA). Cette altération a été observée dans le groupe de population arabe portant l'haplotype fondateur ARA2. Cette altération est donc caractéristique de l'haplotype fondateur ARA2.

La troisième altération de séquence (variation Arm3) consiste en un changement T -> C qui remplace un codon valine (GTT) en codon alanine (GCT). Cette altération a été observée dans le groupe de population Druze portant l'haplotype fondateur D et dans le groupe de population Arménienne portant l'haplotype fondateur ARM3. Cette altération est donc caractéristique de l'haplotype fondateur ARM3/D qui est le même.

- La quatrième altération de séquence (variation Arm2) consiste en un changement G -> C qui remplace un autre codon méthionine (ATG) en codon isoleucine (ATC). Cette altération a été observée dans le groupe de population Arménienne et Turque portant l'haplotype fondateur ARM2. Cette altération est donc caractéristique de l'haplotype fondateur ARM2.

D'autres mutations ont été observées dans d'autres exons du gène de la Marenostrine, mais résultant en des codons synonymes (i.e., codant pour le même acide aminé).

### 8. Contrôles

Différentes analyses contrôles ont été réalisées sur de l'ADN génomique en utilisant soit le système ARMS ("Amplification Refractory Mutation System", ref. 23), soit une combinaison appropriée de couples d'amorces de PCR et d'enzymes de restrictions. Plus particulièrement, la première stratégie a été utilisée pour les variations Med et Ara2 et la seconde pour les variations Arm2 et Arm3. La structure des amorces utilisées et les conditions d'amplification ont été décrites plus haut.

Dans un premier temps, la possibilité que les variants de séquences observés en 7. ci-dessus soient corrélés avec les mutations candidates a été explorée, en étudiant la co-ségrégation de ces variants du gène de la Marenostrine avec la maladie. Dans ce but, des descendants porteurs et non-porteurs d'une famille affectée ont été criblés. Les résultats obtenus montrent que dans tous les pédigrés testés (plus de 40), un des quatre variants de séquences était toujours détecté.

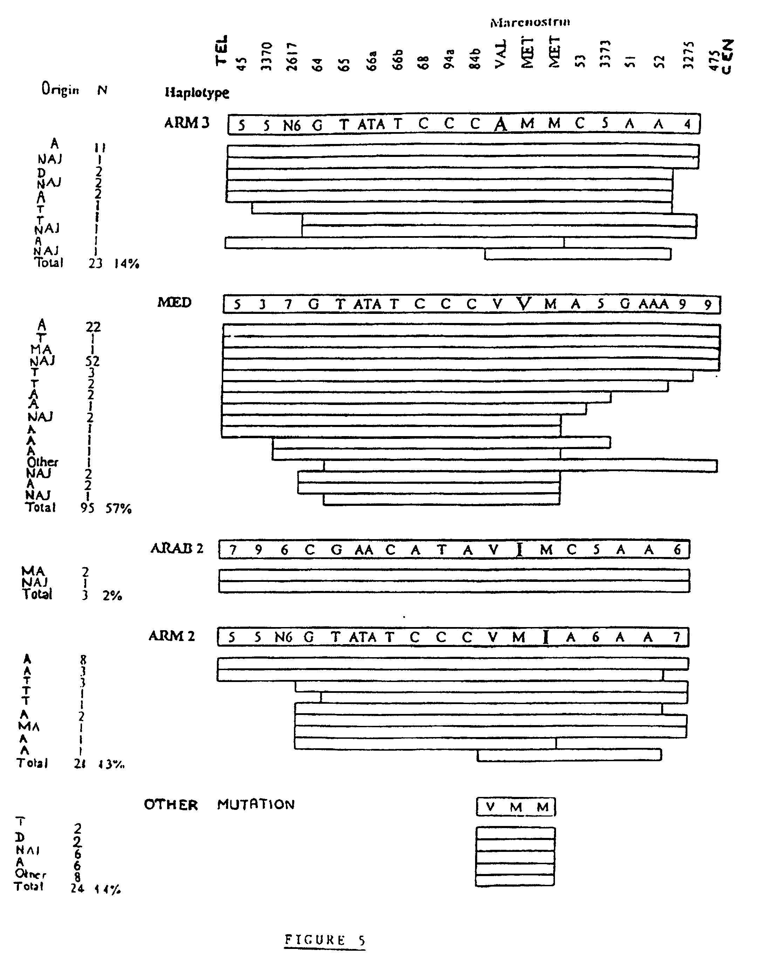

Les variations de séquences ont ensuite été examinées pour un grand nombre de chromosomes porteurs, et chaque altération s'est avérée restreinte à un haplotype fondateur unique (Figure 5). Ainsi, le variant Med (Met -> Val) se retrouve dans 83% des chromosomes porteurs chez les patients Juifs d'Afrique du Nord, en corrélation stricte avec tous les haplotypes MED.

Pour exclure la possibilité que ces variants soient simplement des polymorphismes communs dans la population, un panel d'échantillons d'ADNs de controle a été testé pour la présence de chaque variation. Les résultats obtenus montrent que les variants de séquences n'ont été détectés pour aucun des échantillons d'ADN des 73 sujets testés (146 chromosomes), provenant de familles CEPH de groupes caucasiens divers.

Compte tenu de la fréquence élevée de la mutation dans les populations Juives d'Afrique du Nord et Arméniennes, les contrôles négatifs utilisés ont été restreints aux chromosomes non-porteurs de sujets atteints dans ces populations. Aucune des 4 altérations Med, Ara2, Arm2 et Arm3 n'a été trouvée dans les 162 chromosomes ainsi étudiés. En particulier, aucun des autres chromosomes non-porteurs présentant un haplotype de type MED ne porte la variation Med. De manière similaire, alors que des combinaisons contigües d'allèles correspondant à l'haplotype ARA2 ont été trouvées fréquemment chez des individus non-porteurs, le variant Ara2 (Met -> Ile) n'est présent que dans les chromosomes porteurs.

De manière générale, les résultats obtenus ci-dessus montrent qu'aucun chromosome non-porteur, y compris ceux présentant des homologies aux haplotypes fondateurs, ne porte les variations de séquences décrites ci-avant. Ces variations sont donc strictement corrélées aux haplotypes fondateurs et aux chromosomes porteurs, et sont donc bien spécifiques de la pathologie FMF. En outre, ces mutations présentent une pénétrance très élevée chez les patients atteints, ce qui confirme (i) leur spécificité, (ii) leur implication dans la pathologie (iii) que le gène Marenostrine est le gène de la FMF.

### REFERENCES BIBLIOGRAPHIQUES

1. Daniels, M., Shohat, T., Brenner-Ulman, A. & Shohat, M. Familial Mediterranean Fever: High gene frequency among the non-Ashkenazic and Ashkenazic Jewish populations in Israel. *Am. J. Med. Genet.* **55**, 311-314 (1995).
2.Rogers, D. *et al.* Familial Mediterranean Fever in Armenians: Autosomal recessive inheritance with high gene frequency. *Am. J. Med. Genet.* **34**, 168-172 (1995).
3.Aksentijevich, I. *et al.* Refined mapping of the gene causing Familial Mediterranean Fever, by linkage and homozygosity studies. *Am. J. Hum. Genet.* **53**, 451-461 (1993).
4.Levy, E. *et al.* Linkage disequilibrium mapping places the gene causing Familial Mediterranean Fever close to D16S246. *Am. J. Hum. Genet.* **58,** 523-534 (1996).
5.The French FMF consortium Localization of the Familial Mediterranean Fever (FMF) gene to a 250 kb interval in Non-Ashkenazi Jewish founder haplotypes. *Am. J. Hum. Genet.* **59,** 603-612 (1996).
6.Sood, R. *et al.* Construction of a 1-Mb restriction-mapped cosmid contig containing the candidate region for the Familial Mediterranean Fever locus (MEFV) on chromosome 16p13.3. *Genomics* **42**, 83-95 (1997).
7.Datson, N. *et al.* Scanning for genes in large genomic regions: cosmid-based exon trapping of multiple exons in a single product. *Nucleic Acids Res.* **24**, 1105-1111 (1996).
8.Roach, J., Boysen. C. & Hood, L. Pairwise end sequencing: a unified approach to genomic mapping and sequencing. *Genomics* **26**, 345-353 (1995).
9.Altschul, S., W, G., W. M., EW, M. & Lipman, D. Basic local alignment search tool. *J. Mol. Biol.* **215**, 403-410 (1990).
10.Adams, M. D. *et al.* Initial assessment of human gene diversity and expression patterns based upon 83 million nucleotides of cDNA sequence. *Nature* **377**, 3 S-174 S (1995).
11.Hillier, L. *et al.* Génération and analysis of 280,000 human sequence tags. *Genome Res*. **6**, 807-828 (1996).
12.Xu, Y., Mural, R., Shah, M. & Uberbacher, E. Recognizing exons in genomic sequence using GRAIL II. *In Genetic Engineering: Principles and Methods.* (ed. Setlow, J.) (Plenum Press, 1994).
13.Solovyev, V., Salamov, A. & Lawrence, C. Predicting internal exons by oligonucleotide composition and discriminant analysis of spliceable open reading frames. *Nuc. Acids Res.* 22, 5156-5163 (1994).
14.Kulp, D., Haussier, D., Reese, M. & Eeckman, F. A generalized hidden Markov model for the récognition of human genes in DNA. In *ISBM-96* (ed (AAAI/MIT Press, St-Louis, MO, 1996).
15.Buck, L. & Axel, R. A novel multigene family may encode odorant receptors: a molecular basis for odor recognition. *Cell* **65**, 175-187 (1991).
16.Klug, A. & Rhodes, D. Zinc-fingers: a novel protein motif for nucleic acid recognition. *Trends Biochem Sci* **12**, 464-467 (1987).
17.Takahashi, M. & Cooper, G. Ret transforming gene encodes a fusion protein homologous to tyrosine kinases. *Mol. Cell. Biol.* **7**, 1378-1385 (1987).
18.Jack, J. & Mather, I. Cloning and analysis of cDNA encoding bovine butyrophilin, an apical glycoprotein expressed in mammary tissue and secreted in association with the milk-fat globule membrane during lactation. J. *Biol. Chem.* **265,** 14481-14486 (1990).
19.Bellini, M., Lacroix, J. & Gall, J. A putative zinc-binding protein on lampbrush chromosomes loops. *EMBO J.* **12**, 107-114 (1993).
20.Patarca, R. *et al.* rpt-1, an intracellular protein from helper/inducer T cells that régulâtes gene expression of interleukin 2 receptor and human immunodeficiency virus type 1. *Proc. Natl. Acad. Sci. USA* **85,** 2733-2737 (1988).
21.Tsugu, H., Horowitz, R., Gibson, N. & Frank, M. The location of a disease-associated polymorphism and genomic structure of the human 52-kDa Ro/SSA locus (SSA1). *Genomics* 24, 541-548 (1994).
22.Gouzy, J., Corpet, F. & Kahn, D. Graphical interface for ProDom domain families. *Trends Biochem.* **21**, 493 (1996).
23.Newton, C. *et al*. Analysis of any point mutation in DNA. The amplification refractory mutation system (ARMS). Nucleic Acids Res. 17, 2503-2516 (1989).
24.Dausset, J. *et al*. Program description: Centre d'Etude du polymorphism Humain (CEPH): collaborative genetic mapping of the human genome. *Genomics* 6, 575-578 (1990).
25.Almeida, M. *et al.* Haplotype analysis of common transthyretin mutations. *Hum. Genet*. **96**, 350-354 (1995).
26.Mott, R., Grigoriev, A., Maier, E., Hoheisel, J. & Lehrach, H. Algorithms and software tools for ordering clones libraries: Application to the mapping of the genome of Schizosaccharomyces pombe. *Nucleic* Acids *Res.* **21**, 1965-1974 (1993).
27.Goguel, A., Pulcini, F, Danglot, G. & Fauvet, D. Mapping of 22 YACs on Human Chromosomes by FISH using Yeast DNA Alu PCR products for competition. *Ann. Genet*. **39**, 64-68 (1996).
28.Rosenberg, C. & Florijn, R. High resolution DNA Fiberfish on yeast artificial chromosomes direct visualization of DNA replication. *Nature Genetics* **10**, 477-479 (1995).
29.Wiegant, J. *et al.* High resolution in situ hybridization using DNA halo preparations. *Hum. Mol. Genet.* 1, 587-591 (1992).
30.Rychlik, W. & Rhoads, R. A computer program for choosing optimal oligonucleotides for filter hybridization, sequencing and in vitro amplification of DNA. *Nucleic Acids Res.* 17, 8543-8551 (1989).

## Revendications

1. Acide nucléique isolé codant pour la séquence d'acides aminés correspondant à la séquence SEQ ID 1.

2. Acide nucléique selon la revendication 1, **caractérisé en ce qu'**il porte une mutation au moins, choisie parmi les mutations suivantes :
- A → G (1170)
- G → A (1172)
- T → C (1267)
- G → C (1130)

3. Acide nucléique selon la revendication 1, **caractérisé en ce qu'**il s'agit d'ADNg ou d'ADNc.

4. Acide nucléique selon la revendication 1, **caractérisé en ce qu'**il s'agit d'ARN.

5. Gène humain comprenant la séquence SEQ No. 1.

6. Polypeptide codé par l'acide nucléique objet de l'une des revendications 1 à 4.

7. Polypeptide, **caractérisé en ce qu'**il comprend plus de 10 acides aminés consécutifs de la séquence SEQ ID 1.

8. Polypeptide, **caractérisé en ce qu'**il comprend plus de 10 acides aminés consécutifs de la séquence SEQ ID 1 dont au plus 10 % des résidus sont modifiés.

9. Protéine codée par le gène objet de la revendication 5.

10. Sonde pour la détection du gène responsable de la FMF correspondant à tout ou partie de la séquence SEQ ID 1 ou son brin complémentaire.

11. Sonde pour la détection du gène responsable de la FMF composé de 100 à 700 nucléotides dont la moitié au moins est représentée par un fragment de la séquence SEQ ID 1, le reste étant représenté par la séquence flanquante de la séquence SEQ ID 1 s'étendant depuis le résidu 1433 ou son brin complémentaire.

12. Sonde selon la revendication 10, **caractérisée en ce qu'**elle comprend la séquence comprise entre les résidus 1 000 et 1 400 de la séquence SEQ ID 1 ou son brin complémentaire.

13. Sonde selon la revendication 10, **caractérisée en ce qu'**elle recouvre au moins un résidu muté dans le cas de la FMF choisi dans le groupe comprenant les résidus situés aux positions 1170, 1172, 1174, 1267, 1130 de la séquence SEQ ID 1 ou un résidu délété situé en position 1166 de la séquence SEQ ID 1 ou son brin complémentaire.

14. Sonde selon la revendication 10, **caractérisée en ce qu'**elle recrouvre au moins un résidu non muté choisi dans le groupe comprenant les résidus situés aux positions 1170, 1172, 1174, 1267, 1130 de la séquence SEQ ID 1 ou un résidu non délété situé en position 1166 de la séquence SEQ ID 1 ou son brin complémentaire.

15. Amorce complémentaire d'une partie au moins de la séquence SEQ ID 1 portant l'une au moins des mutations suivantes :
- A → G qui remplace le codon méthionine ATG en position 1170 sur la séquence SEQ ID I en un codon valine GTG (variation Med) ;
- G → A qui remplace le codon méthionine (ATG) en position 1172 sur la séquence SEQ ID 1 en un codon isoleucine (ATA) (variation Ara2);
- T → C qui remplace le codon valine (GTT) en position 1267 sur la séquence SEQ ID 1 en codon alanine (GCT) (variation Arm3) ;
- G → C qui remplace le codon méthionine (ATG) en position 1130 sur la séquence SEQ ID 1 en un codon isoleucine (ARC) (variation Arm2).

16. Amorce complémentaire d'une région de la séquence SEQ ID 1 comprenant au moins l'un des nucléotides 1130, 1170, 1172 et 1267, lesdits nucléotides ne présentant pas de mutations.

17. Couple d'amorces comprenant :
a/ une amorce hybridant, dans des conditions de stringence, une région comprise entre les résidus 1 200 et 1 900, avantageusement 1 300 à 1 600 de la séquence SEQ ID 1,
b/ une amorce hybridant dans des conditions de stringence l'autre brin, dans une région comprise entre les résidus 300 à 1 000, de préférence 500 à 1 000, encore plus préférentiellement 700 à 1 000, de la séquence SEQ ID 1,
c/ ces deux amorces permettant l'amplification d'une région de 500 pb au plus comportant tout ou partie de la région comprise entre les résidus 300 à 1 900 de la séquence SEQ ID 1.

18. Couple d'amorces selon la revendication 17, **caractérisé en ce que** l'une des amorces a/ ou b/ inclut au moins un des résidus mutés en position 1130, 1170, 1172 ou 1267 de la séquence SEQ ID 1.

19. Procédé de détection de la présence d'une mutation dans le gène objet de la revendication 5 susceptible de causer la FMF comprenant :
la détection de la présence d'une ou plusieurs mutations dans un échantillon biologique comprenant un acide nucléique par hybridation dans des conditions de stringence avec une sonde selon l'une quelconque des revendications 10 à 14.

20. Procédé selon la revendication 19, **caractérisé en ce que** l'acide nucléique code les acides aminés correspondant au moins à la région comprise entre les résidus 1130 et 1267 de la séquence SEQ ID 1.

21. Procédé selon l'une des revendications 19 ou 20, **caractérisé en ce qu'**il comprend en outre une étape d'amplification de l'acide nucléique préalable ou concomitante à l'étape b.

22. Procédé selon la revendication 21, **caractérisé en ce que** l'amplification est réalisée en utilisant un couple d'amorces selon la revendication 18 de sorte que l'amplification ne se produit que lorsque la mutation spécifique de l'amorce est présente dans l'acide nucléique.

23. Trousse utilisable pour la mise en oeuvre d'un procédé tel que défini dans l'une des revendications 19 à 22 comprenant au moins un couple d'amorces selon l'une des revendications 17 ou 18.

24. Trousse selon la revendication 23, **caractérisée en ce qu'**elle comprend en outre une sonde telle que définie dans l'une des revendications 10 à 14.

## Claims

1. Isolated nucleic acid encoding the amino sequence of SEQ ID1.

2. Nucleic acid according to Claim 1, **characterized in that** it carries at least one mutation selected from the following mutations:
- A-> G (1170)
- G -> A (1172)
- T -> C (1267)
- G -> C (1130)

3. Nucleic acid according to Claim 1, **characterized in that** it is a gDNA or cDNA.

4. Nucleic acid according to Claim 1, **characterized in that** it is an RNA.

5. Human gene comprising sequence SEQ ID1.

6. Polypeptide encoded in a nucleic acid according to any one of the claims 1 to 4.

7. Polypeptide **characterized in that** it comprises more than two consecutive residues of SEQ ID1.

8. Polypeptide **characterized in that** it comprises more than 10 consecutive residues of SEQ ID1, less than 10 % of the residues of the sequence being affected by modification.

9. Protein encoded by the gene according to claim 5.

10. Probe for the detection of the gene responsible for FMF which is represented by all or part of the sequence SEQ ID1 or its complementary strand.

11. Probe for the detection of the gene responsible for FMF which is composed of 100 to 700 nucleotides, half of which is represented by a fragment of the sequence SEQ ID1, the part which is not, is represented by the flanking sequence of SEQ ID1 from the residue 1433 or its complementary strand.

12. Probe according to claim 10, **characterized in that** it comprises the sequence included between the residue 1 000 and 1 400 of the sequence SEQ ID1 or its complementary strand.

13. Probe according to claim 10, **characterized in that** it covers at least one mutated residue in FMF selected from the group comprising the residues at position 1170, 1172, 1174, 1267, 1130 of the sequence SEQ ID1 or a deleted residue at position 1166 of the sequence SEQ ID1 or its complementary strand.

14. Probe according to claim 10, **characterized in that** it covers at least one non mutated residue selected from the group comprising the residues situated at position 1170, 1172, 1174, 1267, 1130 of the sequence SEQ ID1 or a residue non deleted situated at position 1166 of the sequence SEQ ID1 or its complementary strand.

15. Primer, which is complementary to at least a part of the sequence SEQ ID1, carrying at least one of the following mutations:
- A - > G which replaces the methionine codon ATG at position 1170 in the sequence SEQ ID1 by a valine codon GTG (variation Med);
- G - > A which replaces the methionine codon ATG at position 1172 in the sequence SEQ ID1 by an isoleucine codon ATA (variation Ara2);
- T - > C which replaces the valine codon GTT at position 1267 in the sequence SEQ ID1 by an alanine codon GCT (variation Arm3);
- G - > C which replaces the methionine codon ATG at position 1130 in the sequence SEQ ID1 by an isoleucine codon ATC (variation Arm2).

16. Primer which is complementary to a region of the sequence SEQ ID1 comprising at least on of the nucleotides 1130, 1170, 1172 and 1267, said nucleotides no exhibiting mutation

17. Primer couple comprising:
a) one primer hybridising in stringent conditions a region included between the residues 1 200 to 1 900, advantageously 1 300 to 1 600 of sequence SEQ ID1;
b) one primer hybridising in stringent conditions with the other strand in a region included between the residues 300 to 1 000, preferably 500 to 1 000, more preferably 700 to 1 000 of the sequence SEQ ID1;
c) this primer couple makes possible the amplification of a region of maximally 500 bp comprising all or part of the region included between the residues 300 to 1 900 of the sequence SEQ ID1.

18. Primer couple according to Claim 17, **characterized in that** one of the primers a) or b) includes at least one of the mutated residues at positions 1130, 1170, 1172 or 1267 of the sequence SEQ ID1.

19. A method for the detection of the presence of a mutation in the gene according to claim 5 capable of causing FMF comprising the detection of the presence of one or more mutations in a biological sample containing a nucleic acid by hybridisation in stringent conditions with a probe according to Claims 10-14.

20. Method according to Claim 19, **characterized in that** the nucleic acid codes at least for the residues of the region included between the residues 1130 and 1267 of the sequence SEQ ID1.

21. Method according to one of Claims 19 or 20, **characterized in that** it comprises in addition an amplification step for the nucleic acid, prior to or concomitant with step b).

22. Method according to Claim 20, **characterized in that** the amplification is performed by using a primer couple according to Claim 18, such that the amplification only occurs when the specific mutation of the primer is present in the nucleic acid.

23. Kit usable for the implementation of a method such as that defined in one of Claims 19 to 22 comprising at least one primer couple according to one of claims 17 or 18.

24. Kit according to Claim 23, **characterized in that** it further comprises a probe according to one of claims 10 to 14.

## Patentansprüche

1. Isolierte Nukleinsäure, die für die der Sequenz SEQ ID 1 entsprechenden Aminosäurensequenz kodiert.

2. Nukleinsäure nach Anspruch 1, **dadurch gekennzeichnet, dass** sie mindestens eine Mutation aufweist, ausgewählt aus den folgenden Mutationen:
- A → G (1170)
- G → A (1172)
- T → C (1267)
- G → C (1130)

3. Nukleinsäure nach Anspruch 1, **dadurch gekennzeichnet, dass** es sich um gDNA oder cDNA handelt.

4. Nukleinsäure nach Anspruch 1, **dadurch gekennzeichnet, dass** es sich um RNA handelt.

5. Humanes Gen, umfassend die Sequenz SEQ ID 1.

6. Polypeptid, das durch die Nukleinsäure nach einem der Ansprüche 1 bis 4 kodiert wird.

7. Polypeptid, **dadurch gekennzeichnet, dass** es mehr als 10 aufeinanderfolgende Aminosäuren der Sequenz SEQ ID 1 umfasst.

8. Polypeptid, **dadurch gekennzeichnet, dass** es mehr als 10 aufeinanderfolgende Aminosäuren der Sequenz SEQ ID 1 umfasst, wovon höchstens 10 % der Reste modifiziert sind.

9. Protein, kodiert durch das Gen nach Anspruch 5.

10. Sonde zum Nachweis des für FMF verantwortlichen Gens, die der gesamten oder einem Teil der Sequenz SEQ ID 1 oder ihrem komplementären Strang entspricht.

11. Sonde zum Nachweis des für FMF verantwortlichen Gens, das aus 100 bis 700 Nukleotiden besteht, wovon mindestens die Hälfte durch ein Fragment der Sequenz SEQ ID 1 oder dem komplementären Strang dargestellt wird, wobei der Rest.durch die flankierende Sequenz der Sequenz SEQ ID 1 dargestellt wird, die sich von dem Rest 1433 erstreckt.

12. Sonde nach Anspruch 10, **dadurch gekennzeichnet, dass** sie die Sequenz umfasst, die zwischen den Resten 1000 und 1400 der Sequenz SEQ ID 1 oder ihren komplementären Strang liegt

13. Sonde nach Anspruch 10, **dadurch gekennzeichnet, dass** sie mindestens einen mutierten Rest im Fall von FMF erkennt, der ausgewählt ist aus der Gruppe, bestehend aus den Resten, die sich in den Positionen 1170, 1172, 1174, 1267, 1130 der Sequenz SEQ ID 1 befinden, oder einen deletierten Rest erkennt, die sich in Position 1166 der Sequenz SEQ ID1 befindet, oder des komplementären Strangs.

14. Sonde nach Anspruch 10, **dadurch gekennzeichnet, dass** sie mindestens einen nicht mutierten Rest erkennt, der ausgewählt ist aus der Gruppe, bestehend aus den Resten, die sich in den Positionen 1170, 1172, 1174, 1267, 1130 der Sequenz SEQ ID 1 befinden, oder einen nicht deletierten Rest, der sich in Position 1166 der Sequenz SEQ ID1 befindet, oder des komplementären Strangs.

15. Primer, der mindestens zu einem Teil der Sequenz SEQ ID 1 komplementär ist, der mindestens eine der folgenden Mutationen aufweist;
- A → G, ersetzt das Kodon Methionin ATG in Position 1170 in der Sequenz SEQ ID 1 durch ein Koden Valin GTG (Variation Med);
- G → A, ersetzt das Kodon Methionin (ATG) in Position 1172 in der Sequenz SEQ ID 1 durch ein Kodon Isoleucin (ATA) (Variation Ara2);
- T → C, ersetzt das Kodon Valin (GTT) in Position 1267 in der Sequenz SEQ ID1 durch ein Kodeon Alanin (GCT) (Variation Arm3);
- G → C, ersetzt das Kodeon Methionin (ATG) in Position 1130 in der Sequenz SEQ ID 1 durch ein Kodeon Isoleucin (ARC) (Variation Arm2).

16. Primer, der zu einem Bereich der Sequenz SEQ ID 1 komplementär ist, der mindestens eines der Nucleotide 1130, 1170, 1172 und 1267 umfasst, wobei die Nukleotide keine Mutationen aufweisen.

17. Primerpaar, umfassend;
a) einen Primen, der unter stringenten Bedingungen an einen Bereich hybridisiert, der zwischen den Resten 1200 und 1900, vorteilhafter Weise 1300 und 1600 der Sequenz SEQ ID 1 liegt,
b) einen Primer, der unter stringenten Bedingungen an den anderen Strang in einem Bereich hybridisiert, der zwischen den Resten 300 bis 1000, vorzugsweise 500 bis 1000, und noch bevorzugter 700 bis 1000 der Sequenz SEQ ID 1 liegt,
c) wobei beide Primer die Amplifikation eines Bereichs von höchstens 500 bp ermöglichen, der den gesamten oder einen Teilbereich umfasst, der zwischen den Resten 300 bis 1900 der Sequenz SEQ ID 1 liegt.

18. Primerpaar nach Anspruch 17, **dadurch gekennzeichnet, dass** einer der Primer a) oder b) mindestens einen mutierten Rest in Position 1130, 1170, 1172 oder 1267 der Sequenz SEQ ID 1 enthält.

19. Verfahren zum Nachweis einer Mutation in dem Gen nach Anspruch 5, die FMF hervorrufen kann, umfassend;
den Nachweis von einer oder mehrerer Mutationen in einer biologischen, Nukleinsäure-haltigen Probe durch Hybridisierung unter stringenten Bedingungen mit einer Sonde nach einem der Ansprüche 10 bis 14.

20. Verfahren nach Anspruch 19, **dadurch gekennzeichnet, dass** die kodierende Nukleinsäure mindestens dem Bereich entspricht, der zwischen den Resten 1130 und 1267 der Sequenz SEQ ID 1 liegt.

21. Verfahren nach einem der Ansprüche 19 oder 20, **dadurch gekennzeichnet, dass** es außerdem einen Schritt der Amplifikation der Nukleinsäure vor Schritt b oder während Schritt b umfasst.

22. Verfahren nach Anspruch 21, **dadurch gekennzeichnet, dass** die Amplifikation durch die Verwendung eines Primerpaars nach Anspruch 18 erfolgt, wobei die Amplifikation nur stattfindet, wenn die spezifische Mutation des Primers in der Nukleinsäure vorhanden ist.

23. Kit zur Durchführen des Verfahrens nach einem der Ansprüche 19 bis 22, umfassend mindestens ein Primerpaar nach einem der Ansprüche 17 oder 18.

24. Kit nach Anspruch 23, **dadurch gekennzeichnet, dass** des weiteren eine Sonde nach einem der Ansprüche 10 bis 14 darin enthalten ist.
